# EUROPEAN PATENT APPLICATION

(11) **EP 2 163 563 A1**
(43) Date of publication of application: **17.03.2010**
(21) Application number: 09014569.9
(22) Date of filing: 29.03.2007
(51) Int. Cl.: C07K 16/28

(54) **Treatment of tumors expressing mutant EGF receptors**

(30) Priority: 31.03.2006 US 788426 P
(62) Divisional of application: 07754405.4
(71) Applicant: Massachusetts Institute of Technology, Cambridge, MA 02139 (US); EMD Lexigen, Billerica, MA 01821 (US)
(72) Inventor: Way, Jeffrey C., Cambridge, MA 02138 (US); Chao, Ginger, Somervilee, MA 02143 (US); Cresson, Catherine, New Orleans, LA 70124 (US); Lauffenburger, Douglas, Cambridge, MA 02139 (US); Wittrup, K. Dane, Chestnut Hill, MA 02467 (US)
(74) Representative: Bösl, Raphael Konrad

(57) **Abstract**

The invention discloses methods of treatment of tumors that express oncogenic forms of the epidermal growth factor receptor (EGFR), such as EGFRvIII. The methods include testing of cancer patients for expression of EGFRvIII in their tumors, followed by treatment with a protein that contains antibody variable regions that recognize specific epitopes on the surface of the EGFR.

SEQUENCE LISTING

<110>  MASSACHUSETTS INSTITUTE OF TECHNOLOGY et al.

<120>  Treatment of Tumors Expressing Mutant EGF Receptors

<130>  C65998PCEPT1

<150>  US 60/788,426
<151>  2006-03-31

<160>  26

<170>  PatentIn version 3.3

<210>  1
<211>  112
<212>  PRT
<213>  Artificial

<220>
<223>  synthetic

<400>  1

Asp Val Leu Met Thr Gln Ile Pro Leu Ser Leu Pro Val Ser Leu Gly
1               5                   10                  15

Asp Gln Ala Ser Ile Ser Cys Arg Ser Ser Gln Asn Ile Val His Ser
            20                  25                  30

Asn Gly Asn Thr Tyr Leu Asp Trp Tyr Leu Gln Lys Pro Gly Gln Ser
        35                  40                  45

Pro Asn Leu Leu Ile Tyr Lys Val Ser Asn Arg Phe Ser Gly Val Pro
    50                  55                  60

Asp Arg Phe Arg Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Tyr
                85                  90                  95

Ser His Val Pro Trp Thr Phe Gly Gly Gly Thr Lys Leu Glu Ile Lys
            100                 105                 110

<210>  2
<211>  106
<212>  PRT
<213>  Artificial

<220>
<223>  synthetic

<400>  2

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Thr Tyr Met
            20                  25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Ser His Ile Phe Thr
                85                  90                  95

Phe Gly Ser Gly Thr Lys Leu Glu Ile Lys
            100                 105

<210>  3
<211>  106
<212>  PRT
<213>  Artificial

<220>
<223>  synthetic

<400>  3

<210>  4
<211>  107
<212>  PRT
<213>  Artificial

<220>
<223>  synthetic

<400>  4

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Ile Lys
            100                 105

<210>  5
<211>  107
<212>  PRT
<213>  Artificial

<220>
<223>  synthetic

<400>  5

Gln Ser Val Leu Thr Gln Pro Pro Ser Leu Ser Val Ser Pro Gly Gln
1               5                   10                  15

Thr Ala Ser Ile Thr Cys Ser Gly Asp Lys Leu Gly Asp Lys Tyr Ala
            20                  25                  30

Ser Trp Tyr Gln Gln Lys Pro Gly Gln Ser Pro Val Leu Val Ile Tyr
        35                  40                  45

Gln Asp Arg Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Pro Tyr
                85                  90                  95

Val Phe Gly Thr Gly Thr Lys Val Thr Val Leu
            100                 105

<210>  6
<211>  107

<212>  PRT
<213>  Artificial

<220>
<223>  synthetic

<400>  6

Gln Ser Ala Leu Thr Gln Pro Pro Ser Val Ser Val Ser Pro Gly Gln
1               5                   10                  15

Thr Ala Ser Ile Thr Cys Ser Gly Asp Lys Leu Gly Asp Lys Tyr Ala
            20                  25                  30

Ser Trp Tyr Gln Leu Lys Pro Ala Gln Ser Pro Val Trp Val Ile Tyr
        35                  40                  45

Gln Asp Thr Lys Arg Ser Ser Gly Ile Pro Glu Arg Ile Ser Gly Ser
    50                  55                  60

Asn Ser Gly Asn Thr Ser Thr Leu Thr Ile Thr Gly Thr Gln Ala Met
65                  70                  75                  80

Asp Glu Ala Asp Tyr Tyr Cys Gln Ala Trp Asp Ser Ser Thr Ala Val
                85                  90                  95

Val Phe Gly Gly Gly Thr Lys Leu Thr Val Leu
            100                 105

<210>  7
<211>  122
<212>  PRT
<213>  Artificial

<220>
<223>  synthetic

<400>  7

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Asn Tyr
            20                  25                  30

Tyr Ile Tyr Trp Val Lys Gln Arg Pro Gly Gln Gly Leu Glu Trp Ile
        35                  40                  45

Gly Gly Ile Asn Pro Thr Ser Gly Gly Ser Asn Phe Asn Glu Lys Phe
    50                  55                  60

Lys Thr Lys Ala Thr Leu Thr Val Asp Glu Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Thr Arg Gln Gly Leu Trp Phe Asp Ser Asp Gly Arg Gly Phe Asp Phe
            100                 105                 110

Trp Gly Gln Gly Thr Thr Leu Thr Val Ser
        115                 120

<210> 8
<211> 120
<212> PRT
<213> Artificial

<220>
<223> synthetic

<400> 8

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Ser His
            20                  25                  30

Trp Met His Trp Val Lys Gln Arg Ala Gly Gln Gly Leu Glu Trp Ile
        35                  40                  45

Gly Glu Phe Asn Pro Ser Asn Gly Arg Thr Asn Tyr Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Arg Asp Tyr Asp Tyr Asp Gly Arg Tyr Phe Asp Tyr Trp Gly
            100                 105                 110

Gln Gly Thr Thr Leu Thr Val Ser
        115                 120

<210> 9
<211> 120
<212> PRT
<213> Artificial

<220>
<223> synthetic

<400> 9

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser His
            20                  25                  30

Trp Met His Trp Val Arg Gln Ala Pro Gly Gln Gly Leu Glu Trp Ile
        35                  40                  45

Gly Glu Phe Asn Pro Ser Asn Gly Arg Thr Asn Tyr Asn Glu Lys Phe
    50                  55                  60

Lys Ser Lys Ala Thr Met Thr Val Asp Thr Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Ser Arg Asp Tyr Asp Tyr Asp Gly Arg Tyr Phe Asp Tyr Trp Gly
            100                 105                 110

Gln Gly Thr Leu Val Thr Val Ser
        115                 120

<210>  10
<211>  118
<212>  PRT
<213>  Artificial

<220>
<223>  synthetic

<400>  10

Gln Val Gln Leu Lys Gln Ser Gly Pro Ser Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser
        115

<210>  11
<211>  123
<212>  PRT

<213>  Artificial

<220>
<223>  synthetic

<400>  11

Glu Val Gln Leu Val Glu Ser Gly Gly Gly Leu Val Gln Pro Gly Gly
1               5                   10                  15

Ser Leu Arg Leu Ser Cys Ser Ala Ser Gly Phe Thr Phe Ser Ser Tyr
            20                  25                  30

Ala Met His Trp Val Arg Gln Ala Pro Gly Lys Gly Leu Glu Tyr Val
        35                  40                  45

Ser Ala Ile Ser Ser Asn Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Ser Gln Met Ser Ser Leu Arg Ala Glu Asp Thr Ala Val Tyr Tyr Cys
                85                  90                  95

Phe Lys Asp Val Gly Gly Ser Ser Trp Tyr Trp Ala Asp Tyr Phe Asp
            100                 105                 110

Tyr Trp Gly Gln Gly Thr Leu Val Thr Val Ser
        115                 120

<210>  12
<211>  125
<212>  PRT
<213>  Artificial

<220>
<223>  synthetic

<400>  12

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Arg Leu Ser Glu Asp Thr Ala Val Tyr Tyr Cys
                85                  90                  95

Ala Arg Asp Pro Asp Tyr Tyr Gly Ser Gly Ser Tyr Tyr Pro Asn Trp
            100                 105                 110

Phe Asp Pro Trp Gly Gln Gly Thr Leu Val Thr Val Ser
        115                 120                 125

<210>  13
<211>  31
<212>  DNA
<213>  Artificial

<220>
<223>  synthetic

<400>  13
gtcgggctct ggaggaaaag aaaggtaatt a                                    31

<210>  14
<211>  30
<212>  DNA
<213>  Artificial

<220>
<223>  synthetic

<400>  14
gagtcgggct ctggaggaaa agaaaggtaa                                      30

<210>  15
<211>  25
<212>  DNA
<213>  Artificial

<220>
<223>  synthetic

<400>  15
atcccagtgg cgatggacgg gatct                                           25

<210>  16
<211>  30
<212>  DNA
<213>  Artificial

<220>
<223>  synthetic

<400>  16
ggttttctga ccggaggtcc caaacagttt                                      30

<210>  17
<211>  9
<212>  PRT
<213>  Artificial

<220>
<223>  synthetic

<400>  17

Glu Glu Glu Glu Tyr Phe Glu Leu Val
1               5

<210>  18
<211>  13
<212>  PRT
<213>  Artificial

<220>
<223>  synthetic

<400>  18

Leu Glu Glu Lys Lys Gly Asn Tyr Val Val Thr Asp His
1               5                   10

<210>  19
<211>  5
<212>  PRT
<213>  Artificial

<220>
<223>  synthetic

<400>  19

Leu Glu Glu Lys Lys
1               5

<210>  20
<211>  7
<212>  PRT
<213>  Artificial

<220>
<223>  synthetic

<400>  20

Asn Tyr Val Val Thr Asp His
1               5

<210>  21
<211>  26
<212>  PRT
<213>  Artificial

<220>
<223>  synthetic

<400>  21

Cys Asn Gly Ile Gly Ile Gly Glu Phe Lys Asp Ser Leu Ser Ile Asn
1               5                   10                  15

Ala Thr Asn Ile Lys His Phe Lys Asn Cys
            20                  25

<210>  22
<211>  373
<212>  DNA
<213>  Artificial

<220>
<223>  synthetic

<400>  22
caggtgaagc tgcagcagtc tggaggaggc ttggtgaagc ctggagcttc actcaaactc      60

tcttgtgtga cttctggatt cacttttcgc aaattcggaa tgtcttgggt ccgccagact     120

tctgacaagt gtcttgagtg ggttgctagt attagtacag gtggttacaa cacctactat     180

agtgacaatg tgaaggggcg attcaccatc tccagagaga atgccaagaa caccctgtac     240

ctgcaaatga gcagtctgaa atctgaggac acggccctgt attactgtac gagggggtat     300

tcaccctact catacgctat ggactactgg ggtcaaggaa ccaccgtcac cgtctctggg     360

atcgagggcc gcg                                                        373

<210>  23
<211>  322
<212>  DNA
<213>  Artificial

<220>
<223>  synthetic

<400>  23
gacattgagg ctacacagtc tcctgcttcc ttatctgtag ctaccggtga gaaagttact      60

atcagatgca tgactagcac tgacattgat gatgatatga actggtatca acagaaaccc     120

ggtgagccac ccaaattcct catctccgag ggaaacactc tcaggcctgg ggttccgtcc     180

cgctttagta gtagtgggac tgggacagac ttcgttttca ccatcgagaa cactctctcc     240

gaggacgtgg gggattacta ctgcttgcag tcctggaacg tcccattaac attcggctgc     300

gggacaaagt tggaaataaa ac                                              322

<210>  24
<211>  15
<212>  PRT
<213>  Artificial

<220>
<223>  synthetic

<400>  24

Leu Glu Glu Lys Lys Gly Asn Tyr Val Val Thr Asp His Gly Cys
1               5                   10                  15


<210>  25
<211>  10
<212>  PRT
<213>  Artificial

<220>
<223>  synthetic

<400>  25

Lys Lys Gly Asn Tyr Val Val Thr Asp His
1               5                   10

<210>  26
<211>  46
<212>  PRT
<213>  Artificial

<220>
<223>  synthetic

<400>  26

Ile Ile Ser Gly Asn Lys Asn Leu Cys Tyr Ala Asn Thr Ile Asn Trp
1               5                   10                  15

Lys Lys Leu Phe Gly Thr Ser Gly Gln Lys Thr Lys Ile Ile Ser Asn
            20                  25                  30

Arg Gly Glu Asn Ser Cys Lys Ala Thr Gly Gln Val Cys His
        35                  40                  45

## Description

### Background of the Invention

In general, the invention features methods of treatment for tumors that express mutant forms of the epidermal growth factor receptor (EGFR).

Cancer is still largely an unsolved problem. In many types of cancers, particularly solid tumors, EGFR is aberrantly expressed, often in a mutant or altered form, and is involved in the pathogenesis of the cancer.

The EGFR gene is the cellular homolog of the erb B oncogene originally identified in avian erythroblastosis viruses. EGFRvIII is a variant of EGFR that is present in many cancers. EGFRvIII is a constitutively active form of the receptor that is the result of a deletion of a 267 amino acid sequence in the extracellular domain.

Two strategies have been used in treating EGFR-driven tumors: monoclonal antibodies, and related proteins, that bind to the extracellular domain, and small molecules that bind to the intracellular kinase domain of EGFR. The effectiveness of the monoclonal antibodies is due in part to the inhibition of receptor signaling and in part to the immunological effects of the antibody on the tumor, such as antibody-dependent cell-mediated cytotoxicity (ADCC). In addition, therapeutic monoclonal antibodies can also affect the internalization and stability of the receptor. Inhibition of signaling appears to be important for the anti-tumor effect of the antibody therapeutic as antibodies that simply recognize tumor-specific antigens are generally ineffective.

Although several anti-EGFR antibodies have been tested and used as cancer therapeutics, there is currently no clear correlation between a patient's response to a particular anti-EGFR antibody and the expression of a particular mutant or wild-type EGFR. Thus, there is a need in the art for anti-cancer treatments that can specifically inhibit signaling of EGFRvIII, and related mutant forms of EGFR, in cancers cells expressing EGFRvIII.

### Summary of the Invention

In general, the invention focuses on the discovery that antibodies recognizing defined epitopes within EGFR are useful for inhibiting signal transduction by both intact EGFR and variants of EGFR, specifically deletion variants of EGFR such as EGFRvIII.

We have discovered the epitope of the anti-EGFR antibody EMD72000. This epitope includes the amino acids Ser460/Gly461 and surrounding amino acids on the surface of EGFR. We have also discovered that binding to this epitope in EGFRvIII by EMD72000, and other antibodies recognizing the same epitope, blocks the dimerization of EGFRvIII with a second EGFR molecule such as intact EGFR or a second EGFRvIII. This is in contrast to antibodies, such as 13A9 and others, which bind to EGFRvIII in a manner that does not sterically inhibit EGFRvIII from adopting a dimerization-competent conformation. We have also discovered that EMD72000 can inhibit the receptor signaling of both EGFRvIII and EGFR.

Accordingly, we have discovered that EMD72000, and other antibodies that bind to the Ser460/Gly461 epitope or that have the ability to block receptor signaling by both EGFR and EGFRvIII, are useful for the treatment of cancers that express or have an increased likelihood of expressing EGFRvIII.

Generally, the invention features methods for treatment of cancers that may express EGFR variants with a deletion in the extracellular domain. The methods generally include the steps of first evaluating the likelihood that a given cancer in a subject expresses a deleted EGFR variant, and then treating the subject with an antibody of the invention. Treatment with such an antibody is preferably performed when the likelihood that the cancer in question expresses a deleted EGFR variant is considered to be high.

In a first aspect, the invention features a method for treating, preventing, or stabilizing a cancer in a subject in need thereof that includes the steps of (a) determining if the cancer expresses EGFRvIII and (b) administering to a subject, determined to have a cancer that expresses EGFRvIII, an antibody that recognizes the Ser460/Gly461 epitope of human EGFR, wherein the antibody is administered for a time and in an amount sufficient to treat, prevent, or stabilize the cancer in the subject.

In preferred embodiments of this aspect of the invention, the antibody includes a heavy chain variable region amino acid sequence that is at least 90%, 91%, 92%, 93%, 94%, 95%, 96%, 97%, 98%, or 99% identical to the amino acid sequence of SEQ DI NO: 9 or a light chain variable region amino acid sequence that is at least 90%, 91%, 92%, 93%, 94%, 95%, 96%, 97%, 98%, or 99% identical to the amino acid sequence of SEQ DI NO: 3. Desirably, the antibody includes a variable region amino acid sequence that is at least 90%, 91%, 92%, 93%, 94%, 95%, 96%, 97%, 98%, or 99% identical to the amino acid sequence of SEQ ID NOs: 3 and 9. Alternatively or additionally, an antibody that recognizes an epitope comprising amino acids 452, 454, 457, 462, or 463, or amino acids found within the sequences from about 406 to 413 or about 314 to 337 of EGFR can be used. Non-limiting examples of antibodies useful in this aspect of the invention include EMD72000, mAb528, h-R3, mAb 425, and antigen binding fragments of any of the above. Also included are humanized, DeImmunized™, modified (e.g., to increase stability or to reduce the immunogenicity in humans when appropriate) or chimeric derivatives of any of the above antibodies. Antibody V regions of the invention may also be used in the context of fusion proteins, single-chain Fv moieties, Fab fragments, and other genetically engineered constructs.

In additional preferred embodiments, the antibody reduces or prevents signaling (e.g., conversion to active conformation, receptor internalization, receptor dimerization, receptor autophosphorylation, and phosphorylation of a substrate) by intact EGFR or EGFRvIII. Desirably, the signaling is reduced by at least 10%, 20%, 30%, 40%, 50%, 60%, 70%, 80%, 90% or more.

In another aspect, the invention features a method for treating, preventing, or stabilizing a cancer in a subject in need thereof that includes the steps of (a) determining if the cancer expresses EGFRvIII and (b) administering to a subject, determined to have a cancer that expresses EGFRvIII, an antibody that binds EGFRvIII and reduces or inhibits receptor signaling by EGFRvIII, wherein the antibody is administered for a time and in an amount sufficient to treat, prevent, or stabilize the cancer in the subject. Desirably, the antibody binds to an epitope comprising amino acids 452, 454, 457, 462, or 463, amino acids Ser460/Gly461 of human EGFR, amino acids 406 to 413 of human EGFR, amino acids 314 to 337 of human EGFR or binds to a site on domain 3 of EGFR that prevents domain 2 from assuming a signaling conformation.

In preferred embodiments of this aspect of the invention, the antibody includes a heavy chain variable region amino acid sequence that is at least 90%, 91%, 92%, 93%, 94%, 95%, 96%, 97%, 98%, or 99% identical to the amino acid sequence of SEQ DI NO: 9 or light chain variable region amino acid sequence that is at least 90%, 91%, 92%, 93%, 94%, 95%, 96%, 97%, 98%, or 99% identical to the amino acid sequence of SEQ DI NO: 3. Desirably, the antibody includes a variable region amino acid sequence that is at least 90%, 91 %, 92%, 93%, 94%, 95%, 96%, 97%, 98%, or 99% identical to the amino acid sequence of SEQ ID NOs: 3 and 9. Non-limiting examples of antibodies useful in this aspect of the invention include EMD72000, mAb528, h-R3, mAb 425, and antigen binding fragments, humanized or chimeric derivatives of any of the above. Also included are humanized, DeImmunized™, modified (e.g., to increase stability or to reduce the immunogenicity in humans when appropriate), or chimeric derivatives of any of the above antibodies. Antibody V regions of the invention may also be used in the context of fusion proteins, single-chain Fv moieties, Fab fragments, and other genetically engineered constructs.

Receptor signaling by EGFRvIII includes conversion to active conformation, receptor internalization, receptor dimerization, receptor autophosphorylation, and phosphorylation of a substrate. Desirably, the signaling is reduced or inhibited by at least 10%, 20%, 30%, 40%, 50%, 60%, 70%, 80%, 90% or more.

Non-limiting examples of cancers that can be treated or prevented using the methods of the invention include those described herein and particularly, glioblastoma, medulloblastoma, breast cancer, ovarian cancer, and prostate carcinoma. In one example, the subject is a subject that has a pre-cancerous lesion that is determined to express EGFRvIII and the method is used to prevent cancer in the subject.

Determining if the cancer expresses EGFRvIII can be carried out by a number of methods. In one example, the type of cancer in the subject is compared to the known percent incidence of EGFRvIII for the same type of cancer in a population to determine the percent likelihood that the cancer expresses EGFRvIII. A cancer with a percent likelihood of at least 57%, 60%, 65%, 70%, 75%, 80%, 85%, 90%, 95% or more is preferred for the methods of the invention.

In another example, a biological sample is obtained from the subject and used to determine the presence of an EGFRvIII polypeptide. If an EGFRvIII polypeptide is detected in the biological sample, the cancer is considered to express EGFRvIII. Preferably, an EGFRvIII binding polypepitde (e.g., an antibody) or antiserum that specifically recognizes the EGFRvIII novel peptide junction is used to detect the EGFRvIII polypeptide. For example, the antibody MR1-1 may be used.

In yet another example, a biological sample is obtained from the subject and used to determine the presence of an EGFRvIII nucleic acid. If an EGFRvIII nucleic acid is detected in the biological sample, the cancer is considered to express EGFRvIII. There are a number of methods known in the art to detect a nucleic acid in a biological sample, several of which are included herein. For example, a nucleic acid that hybridizes to the EGFRvIII mRNA novel peptide junction, or its corresponding cDNA, can be used as a probe to detect the EGFRvIII nucleic acid in a Southern blot, northern blot, or RNase protection assay. A nucleic acid that hybridizes to EGFRvIII can also be used as a primer to detect EGFRvIII mRNA, or its corresponding cDNA, in an amplification based assays such as PCR, RT-PCR, or quantitative real-time PCR. In addition, nucleic acids that hybridize to EGFR can also be used as probes or primers and detection of EGFRvIII can be determined based on the size differences between the detected EGFR DNA or mRNA and EGFRvIII DNA or mRNA, where the presence of EGFRvIII is indicated by a shorter, or faster migrating, band.

The invention also discloses protein complexes that include antibody V regions and portions of EGFR. One preferred embodiment is a complex between EMD72000 and EGFRvIII. Such an EMD72000/EGFRvIII complex may be on the surface of a cultured cell or on the surface of cells in a cancer patient.

By "EGFR" or "intact EGFR" is meant any mammalian mature full-length epidermal growth factor receptor, including human and non-human forms. The 1186 amino acid human EGFR is described in Ullrich et al., Nature 309:418-425 (1984)) and GenBank Accession No. AAH94761.

By "EGFRvIII" is meant a variant of EGFR in which exons 2 through 7 are deleted resulting in a 267 amino acid in-frame deletion in the extracellular domain of EGFR. EGFRvIII is also known as type III mutant, delta-EGFR, EGFRde2-7, and ΔEGFR and is described in U.S. Patent Nos. 6,455,498, 6,127,126, 5,981,725, 5,814,317, 5,710,010, 5,401,828, and 5,212,290. Generally, the mature EGFRvIII protein begins with the following amino acid sequence Leu Glu Glu Lys Lys Gly Asn Tyr Val Val Thr Asp His (SEQ ID NO: 18) and continues with the remainder of mature EGFR onward. Specifically, the first five amino acids of mature EGFR (Leu Glu Glu Lys Lys (SEQ ID NO: 19)) are present, followed by a glycine that results from a hybrid codon due to alternative splicing, and then followed further by the mature EGFR sequence beginning at amino acid 274. Expression of EGFRvIII may result from a chromosomal deletion, and may also result from aberrant alternative splicing. See Sugawa et al., Proc. Natl. Acad. Sci. 87:8602-8606 (1990).

By "EGFRvIII novel peptide junction" is meant the following amino acid sequence Leu Glu Glu Lys Lys Gly Asn Tyr Val Val Thr Asp His (SEQ ID NO: 18) and continuing with the remainder of mature EGFR onward, where the sequence Leu Glu Glu Lys Lys (SEQ ID NO: 19) derives from exon 1 of EGFR, Gly is a novel amino acid deriving from novel codon formed at the splice junction between exon 1 and exon 8, and Asn-Tyr-Val-Val-Thr-Asp-His (SEQ ID NO: 20) and subsequent amino acids are derived from exon 8 of EGFR.

By "a deleted EGFR variant" is meant a deletion variant of EGFR in which a portion of the extracellular domain is missing, such that EGFR signaling becomes at least partially ligand-independent.

By "antibody" is meant an immunoglobulin protein (or proteins such as in the case of a polyclonal antibody), or derivative thereof, whether naturally or synthetically produced, which is capable of binding to an antigen. The term also includes monoclonal antibodies, polyclonal antibodies, multispecific antibodies (e.g. bispecific antibodies) formed from at least two intact antibodies, antibody fragments, and heteroantibodies, which are composed of two or more antibodies, or fragments thereof, of different binding specificity which are linked together. Derivatives within the scope of the term include antibodies that have been modified in sequence, but remain capable of specific binding to an antigen, including interspecies, bispecific, chimeric, humanized antibodies, and immunoconjugates conjugating the antibody to an additional molecule (e.g., a cytotoxic molecule), examples of which are described in PCT Publication No. WO 2004/032960. An antibody may be monoclonal or polyclonal, and present in a variety of media including, but not limited to, serum or supernatant, or in purified form. As used herein, antibodies can be produced by any known technique, including harvest from cell culture of native B lymphocytes, hybridomas, recombinant expression systems, by phage display, or the like. Methods for making monoclonal antibodies are known in the art and include the hybridoma method described by Kohler and Milstein (Nature 256,495 (1975)) and in "Monoclonal Antibody Technology, The Production and Characterization of Rodent and Human Hybridomas" (Burdon et al., Eds, Laboratory Techniques in Biochemistry and Molecular Biology, Volume 13, Elsevier Science Publishers, Amsterdam (1985)), or may be made by well known recombinant DNA methods (see, e. g., U.S.P.N. 4,816, 567). Monoclonal antibodies may also be isolated from phage antibody libraries using the techniques described in Clackson et al., Nature, 352: 624-628 (1991) and Marks et al., J. Mol. Biol., 222: 58, 1-597 (1991), for example. Methods of producing polyclonal antibodies are known to those of skill in the art. For example, polyclonal antibodies can be prepared by immunizing rabbits or other animals by injecting antigen followed by subsequent boosts at appropriate intervals. The animals are bled and sera assayed against purified protein usually by ELISA or by bioassay based upon the ability to block the action of the corresponding gene. When using avian species, e.g., chicken, turkey and the like, the antibody can be isolated from the yolk of the egg. Methods of making chimeric and humanized antibodies are also known in the art. For example, methods for making chimeric antibodies include those described herein and in Chimeric and humanized monoclonal antibodies can be produced by methods known in the art, for example using the techniques described in WO 87/02671; EP 184,187; EP 171,496; EP 173,494; WO 86/01533; U.S. Pat. Nos. 4,816,567, 4,816, 397, 5,585,089, 5,225,539, 6,331,415; EP 125,023; Better et al., Science 240: 1041-1043 (1988); Liu et al., Proc Natl Acad Sci. U.S.A. 84: 3439-3443 (1987); Liu et al., J Immunol. 139: 3521-3526 (1987); Sun et al., Proc Natl Acad Sci. U.S.A. 84: 214-218 (1987); Nishimura et al., Cancer Res. 47: 999-1005 (1987); Wood et al., Nature 314: 446-449 (1985); Shaw et al., J Natl Cancer Inst. 80: 1553-1559 (1988); Morrison Science 229: 1202-1207 (1985); Oi et al., Biotechniques 4: 214 (1986); Jones et al., Nature 321: 552-525 (1986); Verhoeyan et al., Science 239: 1534 (1988); Beidler et al., J Immunol, 141: 4053-4060 (1988); Jones et at., Nature 321: 522-525 (1986); Riechmann et al., Nature 332: 323-327 (1988); and Verhoeyen et al., Science 239: 1534-1536 (1988).

By "antiserum" is meant a human or animal serum containing immunoglobulins that are specific for one or more antigens. Examples of anti-EGFRvIII antiserum are known in the art, for example, as described in Kallio et al., Br. J. Cancer 89:1266-1269 (2003).

"Antibody fragment" or "antibody protein fragment" refers to a portion of an antibody (e.g., Fv) capable of binding to an antigen. Fragments within the scope of the term as used herein include those produced by digestion with various peptidases, such as Fab, Fab' and F(ab)'2 fragments, those produced by chemical dissociation, by chemical cleavage, and by recombinant techniques. Typical recombinant fragments, produced, for example, by phage display, include single chain Fab and scFv ("single chain variable region") fragments. Derivatives within the scope of the term include those that have been modified in sequence, including interspecies, chimeric, and humanized antibodies, but remain capable of binding an antigen.

By "variable region" or "variable domain" is meant the portion of the antibody heavy and light chains having an amino acid sequence that differs extensively in sequence among antibodies and that is used in the binding and specificity of each particular antibody for its particular antigen. The variability is concentrated in the "hypervariable regions" or "complementarity determining regions" (CDRs) both in the light chain and the heavy chain variable domains. The regions between the CDRs are more highly conserved and are called the framework regions (FRs). The variable regions of native heavy and light chains each comprise four FRs (FR1-FR4), largely adopting a β-sheet configuration, connected by three hypervariable regions, which form loops connecting, and in some cases forming part of the 6-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC). The term "hypervariable region" or "CDR" when used herein refers to the amino acid residues of an antibody that are responsible for antigen-binding. Desirably, antibodies useful in the methods of the invention will be substantially identical to at least a part of the variable domain (heavy chain, light chain, or both) of EMD72000.

By "substantially identical" is meant a nucleic acid, protein, or amino acid sequence that shares at least 60%, 65%, 70%, 75%, 80%, 85%, 90%, 95%, 96%, 97%, 98%, 99%, or 100% sequence identity with another nucleic acid, protein, or amino acid sequence. "Substantial identity" may be used to refer to various types and lengths of sequence, such as full-length sequence, epitopes or immunogenic peptides, functional domains, antibody variable regions, coding and/or regulatory sequences, exons, introns, promoters, and genomic sequences. Percent identity between two polypeptides or nucleic acid sequences is determined in various ways that are within the skill in the art, for instance, using publicly available computer software such as Smith Waterman Alignment (Smith and Waterman J. Mol. Biol. 147:195-7, 1981); "BestFit" (Smith and Waterman, Advances in Applied Mathematics, 482-489, 1981) as incorporated into GeneMatcher Plus^{™}, Schwarz and Dayhof "Atlas of Protein Sequence and Structure," Dayhof, M.O., Ed pp 353-358, 1979; BLAST program (Basic Local Alignment Search Tool; (Altschul, S. F., W. Gish, et al., J. Mol. Biol. 215: 403-410, 1990), BLAST-2, BLAST-P, BLAST-N, BLAST-X, WU-BLAST-2, ALIGN, ALIGN-2, CLUSTAL, or Megalign (DNASTAR) software. In addition, those skilled in the art can determine appropriate parameters for measuring alignment, including any algorithms needed to achieve maximal alignment over the length of the sequences being compared. In general, for proteins, the length of comparison sequences will be at least 10 amino acids, preferably 20, 30, 40, 50, 60, 70, 80, 90, 100, 110, 120, 130, 140, 150, 175, 200, 210, 220, 230, 240, 250 amino acids or more, up to the full length of the protein. For nucleic acids, the length of comparison sequences will generally be at least 25, 50, 100, 125, 150, 200, 250, 300, 350, 400, 450, 500, 550, 600, 650, 700, 750, nucleotides or more, up to the full length of the nucleic acid molecule. When comparing identity to an epitope, it is understood that the length of the protein used for comparison will be much shorter, for example, 5, 7, 10, 12, 15, or 20 or more amino acids up to the entire length of the epitope. It is understood that for the purposes of determining sequence identity when comparing a DNA sequence to an RNA sequence, a thymine nucleotide is equivalent to a uracil nucleotide. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

By "epitope" is meant a region on a macromolecule that is recognized by an antibody. Generally, the epitope is in a short region of primary sequence in a protein, and it is generally about 5 to 12 amino acids long, but it can also be a region of secondary structure. Preferred epitopes of the invention include the Ser460/Gly461 epitope of the human EGFR; amino acids 406-413 of the human EGFR; amino acids 314 to 337 of the human EGFR; and any epitope within domain 3 that is within domain 3's interaction surface with amino acids 274-310. For any of the above epitopes, the equivalent epitope in a non-human EGFR is also included and can be determined by alignment of the non-human EGFR with the human EGFR to determine the equivalent residues in the non-human EGFR. Antibodies that bind to the preferred epitopes above can be identified using assays known in the art. In one example, the binding of a candidate antibody to an intact EGFR with serine and glycine at positions 460 and 461, respectively, can be compared to the binding of the candidate antibody to a mutant EGFR in which these positions are replaced with different amino acids, e.g., amino acids with a larger amino side chain. A candidate antibody that binds the wild type EGFR but not the mutant EGFR in this assay is identified as one that binds the Ser460/Gly461 epitope.

By the "Ser460/Gly461 epitope" is meant a region on the surface of EGFR that includes amino acids Ser460 and Gly461, and optionally includes nearby amino acids within about 10 to 15 Angstroms of Ser460 and Gly461. An antibody is said to bind to the Ser460/Gly461 epitope if the binding of the antibody is disrupted by mutations at positions 460 and/or 461 that do not generally disrupt the structure of EGFR. For example, an antibody that binds to human EGFR but does not bind to an otherwise human EGFR in which the positions 460 and 461 are replaced by the murine amino acids proline and asparagine is said to bind to the Ser460/Gly461 epitope. Similarly, an antibody is said to bind to the Ser460/Gly461 epitope if that antibody binds to human EGFR but fails to bind to an otherwise human EGFR in which Ser460 is replaced by an amino acid such as alanine, phenylalanine, proline, threonine, tyrosine, or aspartic acid, or in which Gly461 is, for example, replaced by leucine.

By "cancer" is meant any benign or malignant growth or tumor caused by abnormal and uncontrolled cell division. Examples of cancers include, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenriglioma, schwannoma, meningioma, melanoma, neuroblastoma, and retinoblastoma). Desirably, the methods of the invention are used to treat a cancer that is characterized by EGFRvIII expression.

By "pre-cancerous lesion" is meant a morphologically identifiable lesion from which a malignant tumor is presumed to develop in a significant number of instances.

By "anti-cancer therapy" is meant any therapy intended to prevent, slow, arrest, or reverse the growth of a precancerous lesion, a cancer, or a cancer metastases. Generally, an anti-cancer therapy will reduce or reverse any of the characteristics that define the cancer cell (see Hanahan et al., Cell 100:57-50, 2000). Most cancer therapies target the cancer cell by slowing, arresting, reversing, or decreasing the invasive capabilities, or decreasing the ability of the cell to survive the growth of a cancer cell. Anti-cancer therapies include, without limitation, surgery, radiation therapy (radiotherapy), biotherapy, immunotherapy, chemotherapy, or any combination of these therapies.

By "treating, stabilizing, or preventing cancer" is meant causing a reduction in the size of a tumor, slowing or preventing an increase in the size of a tumor, increasing the disease-free survival time between the disappearance of a tumor and its reappearance, preventing an initial or subsequent occurrence of a tumor, or reducing an adverse symptom associated with a tumor. In one preferred embodiment, the percent of cancerous cells surviving the treatment is at least 20, 40, 60, 80, or 100% lower than the initial number of cancerous cells, as measured using any standard assay. Preferably, the decrease in the number of cancerous cells induced by administration of a therapy of the invention is at least 2, 5, 10, 20, or 50-fold greater than the decrease in the number of non-cancerous cells. In yet another preferred embodiment, the number of cancerous cells present after administration of a therapy is at least 2, 5, 10, 20, or 50-fold lower than the number of cancerous cells present after administration of a placebo or vehicle control. Preferably, the methods of the present invention result in a decrease of 20, 40, 60, 80, or 100% in the size of a tumor as determined using standard methods. Preferably, at least 20, 40, 60, 80, 90, or 95% of the treated subjects have a complete remission in which all evidence of the cancer disappears. Preferably, the cancer does not reappear or reappears after at least 5, 10, 15, or 20 years. In another preferred embodiment, the length of time a patient survives after being diagnosed with cancer and treated with a therapy of the invention is at least 20, 40, 60, 80, 100, 200, or even 500% greater than (i) the average amount of time an untreated patient survives or (ii) the average amount of time a patient treated with another therapy survives.

By "sample" is meant a bodily fluid (e.g., urine, blood, serum, plasma, or cerebrospinal fluid), tissue (e.g., tissue biopsy), or cell in which EGFR is normally detectable.

By "subject" is meant a mammal, including, but not limited to, a human or non-human mammal, such as a bovine, equine, canine, ovine, or feline.

By "percent likelihood" is meant percent probability. In the present invention, the percent likelihood that a cancer expresses EGFRvIII can be determined by comparing the type of cancer in the subject to clinical studies or published reports that list the percent of that same type of cancer that is found to express EGFRvIII. Preferably, for the methods of the invention, the percent likelihood that the cancer expresses EGFRvIII is at least 57%, 60%, 65%, 70%, 75%, 80%, 85%, 90%, 95% or more.

By "receptor signaling" is meant any of the biological activities associated with the EGFR or mutants or derivatives thereof (e.g., EGFRvIII) that lead to the activation of cellular events or cellular pathways. Exemplary biological activities include conversion to active conformation, receptor dimerization (homodimer or heterodimer), receptor autophosphorylation (e.g., at Tyr 992, 1068, 1086, 1148, or or 1173 of human EGFR), substrate phosphorylation, and substrate binding. Substrates of EGFR are known in the art and include Gab1, Shc, EPS8, EPS15, and any polypeptides that include the amino acid sequence EEEEYFELV (SEQ ID NO: 17). Such activities can be measured using assays known in the art or described herein and include kinase assays, immunoassays for receptor substrate binding, structure based analysis of receptor conformation, and receptor internalization assays.

By "reduce" is meant the ability to cause an overall decrease, preferably of 20%, 30%, 40%, 50%, 60%, 70%, 80%, 90% or greater. For example, in one embodiment, antibodies of the invention useful in the treatment methods will cause a reduction of at least 20% in the receptor signaling of EGFRvIII or of both EGFRvIII and EGFR.

Other features and advantages of the invention will be apparent from the following description of the preferred embodiments thereof, and from the claims.

### Brief Description of the Drawings

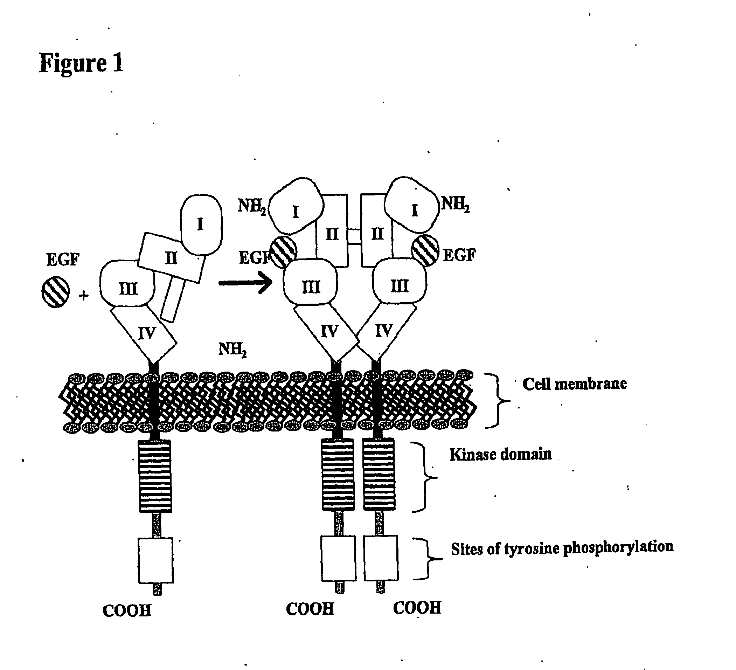

FIGURE 1 schematically depicts the interconversion of the intact EGF receptor in its non-signaling, monomeric state and the signaling, dimeric state that is bound to a ligand such as EGF. The upper portion of the figure shows the extracellular EGFR domains, while the lower portion of the figure shows the intracellular domains, including the kinase domain (horizontal stripes) and an adjacent C-terminal region that is phosphorylated upon dimerization. The left side of the figure shows free Epidermal Growth Factor (EGF; slanted stripes) and unliganded EGFR. A characteristic of the unliganded EGFR is that an extension from Domain 2 interacts with the C-terminal, membrane-proximal region of Domain 4, so that dimerization surfaces in both Domain 2 and Domain 4 are buried. The right side of the figure shows EGFR in the dimeric, liganded state. In this configuration, a ligand such as EGF or TGFα interacts with Domain 1 and Domain 3 of EGFR, while Domain 2 and Domain 4 make dimerization contacts. The interconversion between the active and inactive states of EGFR involves a large-scale movement around the junction of Domains 2 and 3. The result of dimerization is to bring the intracellular kinase domains in proximity to each other and in proximity to substrate sites for tyrosine phosphorylation.

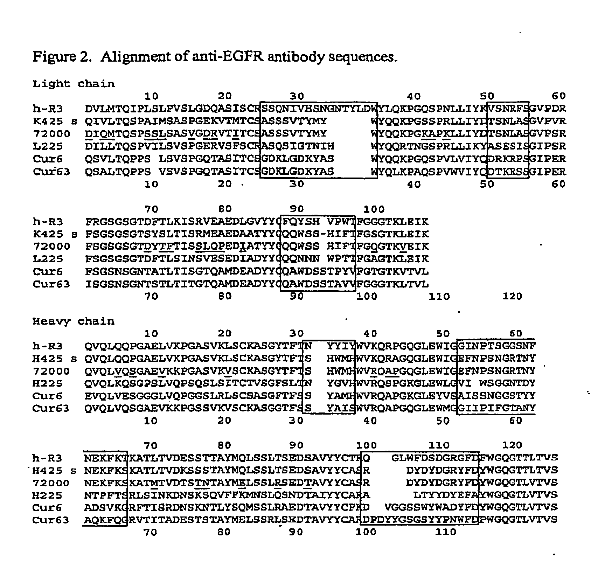

FIGURE 2 shows an alignment of anti-EGFR antibody variable region light chains (SEQ ID NOs: 1-6) and heavy chains (SEQ ID NOs: 7-12). 425 (SEQ ID NO: 2 and 8, for light chain and heavy chain, respectively) is the original mouse monoclonal antibody from which EMD72000 was derived. EMD72000 (SEQ ID NOs: 3 and 9, for light chain and heavy chain, respectively) contains mutations in the framework regions of the 425 variable regions to increase the similarity to human variable regions. H-R3 (SEQ ID NOs: 1 and 7, for light chain and heavy chain, respectively) is an independently isolated antibody that binds to EGFR and is described in U.S. Patent No. 6,506,883. 225 (SEQ ID NOs: 4 and 10, for light chain and heavy chain, respectively) is a mouse monoclonal antibody that binds to an epitope distinct from the 425/EMD72000 epitope. Cur6 (SEQ ID NOs: 5 and 11, for light chain and heavy chain, respectively) and Cur63 (SEQ ID NOs: 6 and 12, for light chain and heavy chain, respectively) are additional anti-EGFR antibodies and are described in U.S. Patent Application Publication No. 20050100546.

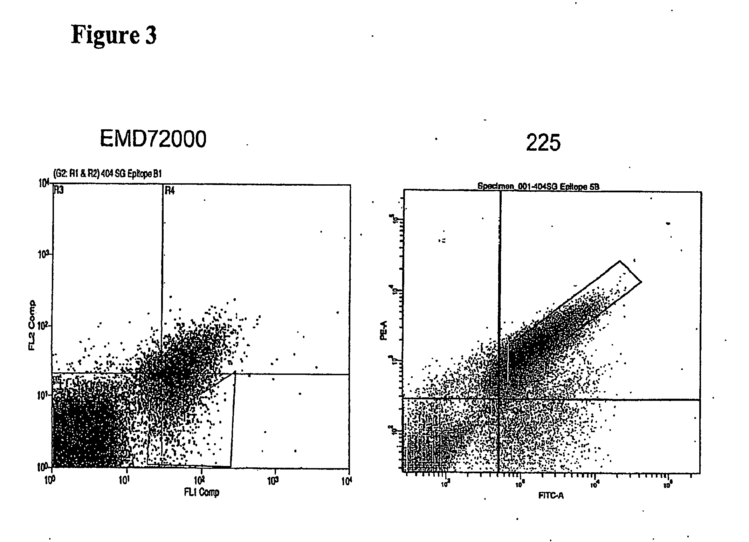

FIGURE 3 shows FACS analyses during the yeast display-based isolation of mutant EGFRs to which EMD72000 does not bind. The left panel shows a population of yeast cells expressing mutagenized EGFR and an epitope tag, to which fluorescently labeled EMD72000 and a differently fluorescently labeled, epitope tag-recognizing antibody have been added. This panel shows cells that have been through four rounds of selection for loss of EMD72000 binding. The Y-axis represents the EMD72000 binding, while the X-axis represents the binding of the antibody recognizing the epitope tag. The cluster of spots at the lower left represents cells that have lost the plasmid expressing EGFR. The cluster of spots toward the center of the panel represent cells to which both EMD72000 and the epitope tag-recognizing antibody are bound in about equal proportion. The spots enclosed in the trapezoid represent cells that appear to retain binding of the epitope tag-recognizing antibody, but have lost binding to EMD72000.

The panel on the right represents a FACS analysis of yeast cells derived from the population taken from within the trapezoid in the left panel. This right panel shows yeast cells to which fluorescently labeled antibody 225 and a differently fluorescently labeled, epitope tag-recognizing antibody have been added. This right panel represents a population that has been through two rounds of selection for retention of antibody 225 binding. The Y-axis represents the 225 binding, while the X-axis represents the binding of the antibody recognizing the epitope tag. The cluster of spots at the lower left again represents cells that have lost the plasmid expressing EGFR. The cluster of spots toward the center of the panel represent cells to which both antibody 225 and the epitope tag-recognizing antibody are bound in about equal proportion. The spots enclosed in the trapezoid represent cells that appear to retain binding of the epitope tag-recognizing antibody and 225, but have lost binding to EMD72000 based on their prior selection.

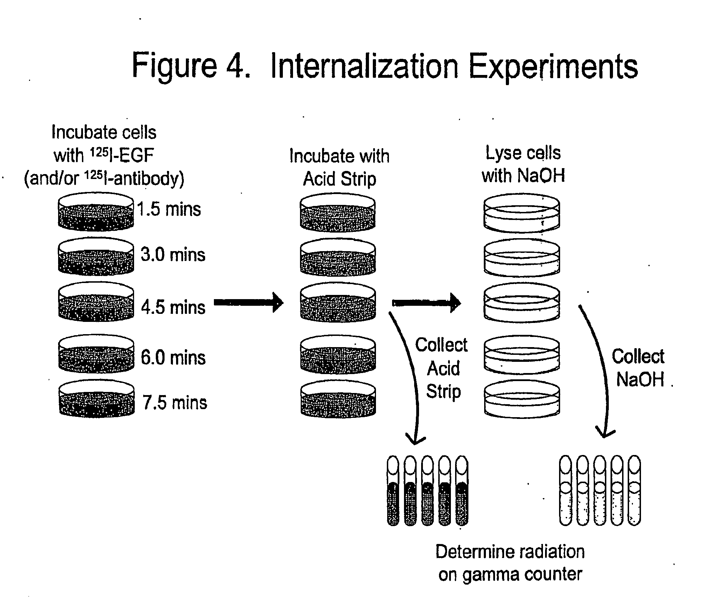

FIGURE 4 illustrates the protocol used to measure the rate of internalization of a radiolabeled protein that binds to EGFR into a cell.

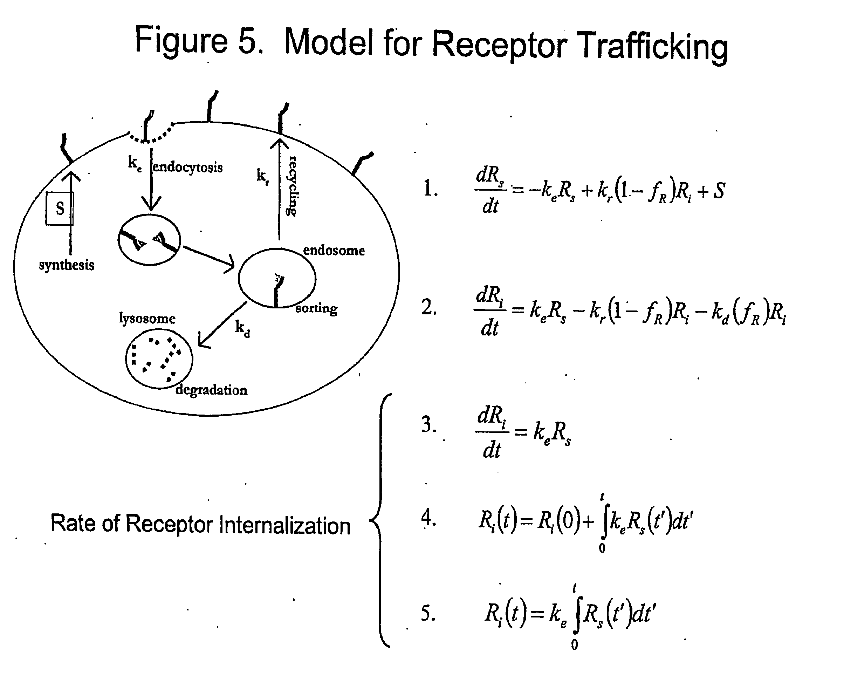

FIGURE 5 schematically illustrates the mathematical model used to calculate the rate of internalization of a radiolabeled protein into a cell.

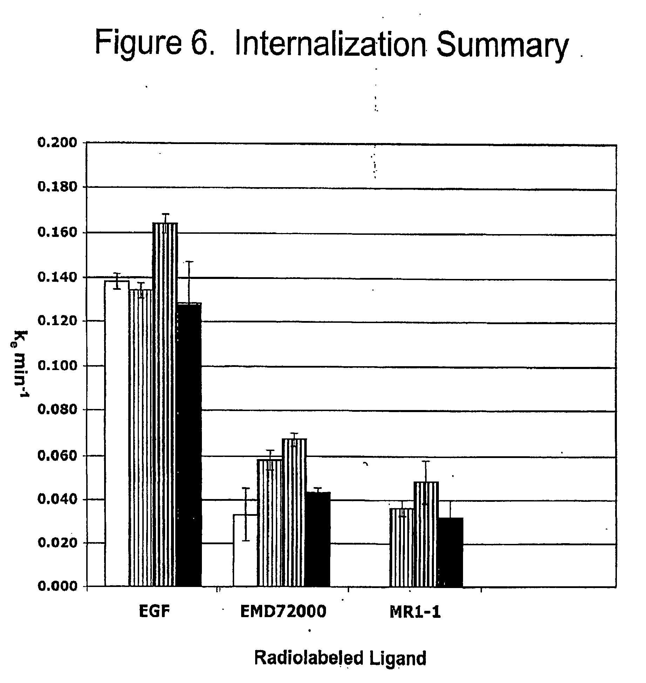

FIGURE 6 is a graph showing the rate of internalization (k_{c}) of radiolabeled EGF, EMD72000, and MR1-1 in immortalized, non-transformed human mammary epithelial cells (HMECs). White bars represent the internalization rates in parental HMECs expressing about 2x10⁵ EGFR per cell, but not EGFRvIII. Bars with thin black stripes, bars with thick black stripes, and black bars represent the internalization rates in transfected HMECs expressing 5x10⁴ EGFRvIII, 5x10⁴ EGFRvIII, 5x10⁴ EGFRvIII, respectively, in addition to expressing 2x10⁵ EGFR per cell.

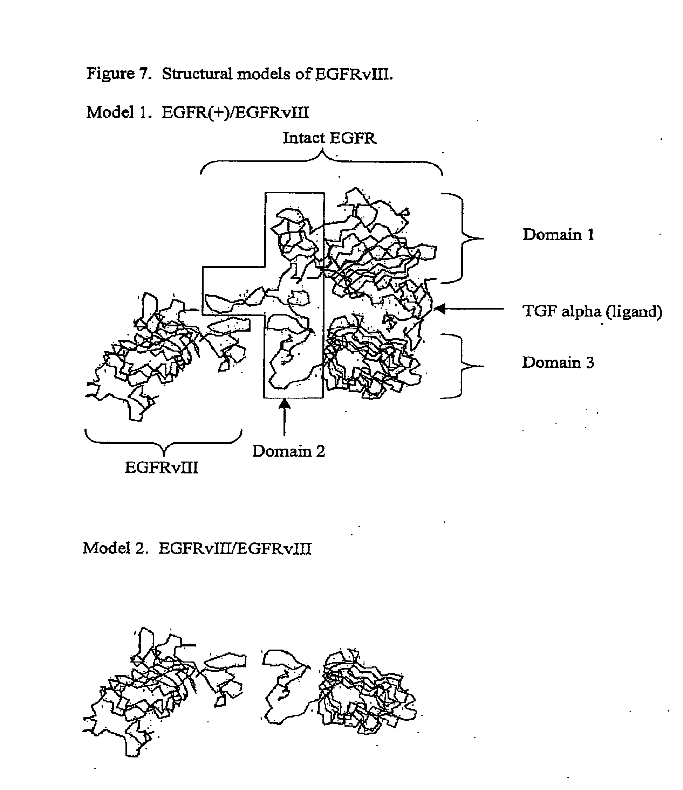

FIGURE 7 shows structural models of an intact EGFR/EGFRvIII heterodimer (top panel) and an EGFRvIII/EGFRvIII homodimer (bottom panel). For simplicity of viewing, side chains have been hidden and only the connected alpha-carbon chain is shown.

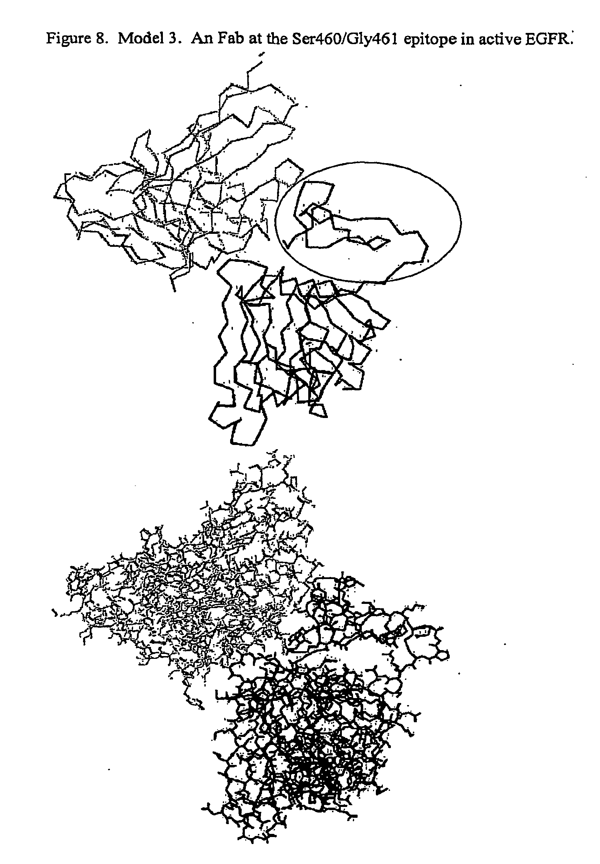

FIGURE 8 shows a structural model of an Fab (gray) positioned over the Ser460/Gly461 epitope in a model of domains 2 and 3 of EGFRvIII in the active conformation. The upper model shows only the alpha carbon trace of the polypeptide backbones. The Fab is in the upper left-hand corner (gray). Domain 3 of EGFR is at the bottom of the figure, and the remnant of Domain 2 present in EGFRvIII is on the right, enclosed in an ellipse. The lower model is the same complex in the same orientation, but now showing all of the backbone and side chain atoms except hydrogen. N-linked oligosaccharides are not shown for ease of viewing.

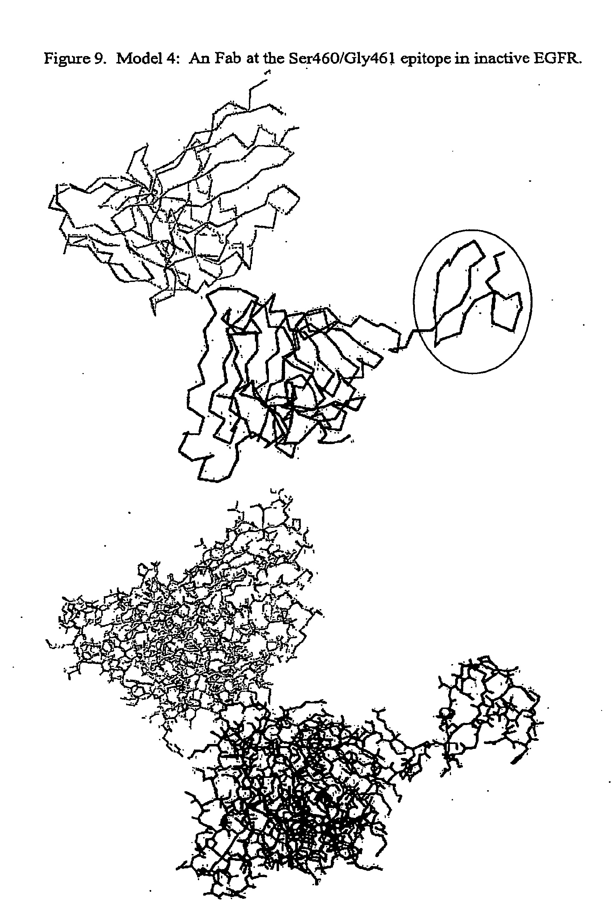

FIGURE 9 shows a structural model of an Fab (gray) positioned over the Ser460/Gly461epitope in a model of domains 2 and 3 of EGFRvIII in the inactive conformation. The upper model shows only the alpha carbon trace of the polypeptide backbones. The Fab is in the upper left-hand corner (gray). Domain 3 of EGFR is at the bottom of the figure, and the remnant of Domain 2 present in EGFRvIII is on the right, enclosed in an ellipse. The lower model is the same complex in the same orientation, but now showing all of the backbone and side chain atoms except hydrogen. N-linked oligosaccharides are not shown for ease of viewing.

### Detailed Description of the Invention

The invention is based on the following discoveries and insights. First, we have discovered that the anti-EGFR antibody EMD72000, a reshaped derivative of monoclonal antibody 425 designed to have reduced immunogenicity, binds to an epitope that includes the amino acids Ser460 and Gly461 and possibly other surrounding amino acids on the surface of EGFR. These results demonstrate that EMD72000 binds an epitope on EGFR that is distinct from the epitopes of 13A9, 225, and 806 (Chao et al., J. Mol. Biol. 342:539-50 (2004); PCT Publication No. WO 2004/032960).

Second, we have discovered that binding to this epitope in EGFRvIII by EMD72000, and other antibodies recognizing the same epitope, blocks the dimerization of EGFRvIII with a second EGFR molecule such as wild-type EGFR or a second EGFRvIII. We have also discovered that EMD72000 can inhibit the receptor signaling of both EGFRvIII and EGFR. The evidence that EMD72000 and other antibodies recognizing the Ser460/Gly461 epitope can block dimerization of EGFRvIII comes from experimental data presented herein, from structural insights based on the known three-dimensional structure of the extracellular domains of EGFR, and structural models of EGFR, EGFRvIII, and EGFR/antibody complexes presented herein.

The present invention features the use of EMD72000, and other antibodies that bind to the Ser460/Gly461 epitope or that have the ability to block receptor signaling by both EGFR and EGFRvIII, for the treatment of cancers that express or have an increased likelihood of expressing EGFRvIII.

The following description of the invention is presented in terms of a specific antibody, EMD72000, but is intended to be illustrative and not limiting. EMD72000 is a humanized version of the murine antibody 425 (U.S. Patent No. 5,558,864). In addition to EMD72000, additional antibodies useful in the methods of the invention include monoclonal antibody 528, h-R3, mAb425. In addition, based on the discovery of the epitope for EMD72000 and the ability of EMD72000 to block the active conformation required for signaling by EGFR and EGFRvIII, it will be apparent to those skilled in the art of protein molecular modeling that additional antibodies useful in the methods of the invention can be identified using the methods described below. Such additional antibodies are included for use in the methods of the invention.

### Preparation of Antibodies Useful in the Methods of the Invention

Antibodies against the extracellular domain of EGFR may be prepared and identified by any of a variety of techniques, such as traditional hybridoma technology, phage display, and others known in the art. Antibodies of the invention can be monoclonal, polyclonal, antigen binding fragments, chimeric antibodies, humanized antibodies, and any derivative that retains the ability to specifically bind the antigen.

Once an antibody that recognizes EGFR is identified, it can be tested for the ability to specifically bind to the epitopes described herein or to specifically inhibit the signaling of EGFR, EGFRvIII, or both using, for example, the methods described herein.

For example, U.S. Patent No. 5,558,864 describes how to make antibodies useful in the methods of the invention.

Once the antibodies are prepared and/or purified, they can be screened for their ability to bind to specific epitopes on EGFRvIII or to inhibit signaling by intact EGFR, EGFRvIII, or both using standard methods known in the art, examples of which are described below.

### Epitope-Based Identification of Antibodies Useful in the Invention

As part of the invention, the binding epitope for EMD72000 was identified and found to include amino acids Ser460/Gly461 of human EGFR. Accordingly, preferred antibodies of the invention can bind to an epitope including these amino acids and/or surrounding amino acids. It should be noted that amino acids can be considered "surrounding" based on primary sequence or secondary structure. In addition, the invention provides the insight that amino acids 406 to 413 or amino acids 314 to 337 are also distinct binding epitopes for antibodies besides EMD72000 that are useful in the methods of the invention. Alternatively or additionally, the epitope can include amino acids 452, 454, 457, 462, or 463. Accordingly, any method used to screen antibodies for their ability to bind any of these epitopes can be used to identify antibodies useful in the methods of the invention.

In one example, the binding of a candidate antibody to a wild-type EGFR with serine and glycine at positions 460 and 461, respectively, can be compared to the binding of the candidate antibody to a mutant EGFR in which these positions are replaced with different amino acids, for example, amino acids with a larger amino side chain. A candidate antibody that binds the wild type EGFR but not the mutant EGFR in this assay is identified as one that binds the Ser460/Gly461 epitope.

In another example, a method for the identification of epitopes using a yeast-display approach as described by Cochran et al. (J. Immunol. Methods. 287:147-58 (2004)) and Chao et al. (J. Mol. Biol. 342:539-50 (2004)) is used. According to this method, a target protein to which an antibody binds is expressed as a fusion protein with the endogenous yeast cell-surface protein Aga2. The presence of the target protein on the yeast cell surface can be detected with a fluorescently labeled antibody, and mutants can be identified in which antibody binding is lost. The mutants are then separated, grown up clonally, and then the mutation(s) in the target protein are identified by DNA sequencing. These mutations are considered to define the epitope for the antibody being tested.

The fusion protein is engineered to have standard epitope tags at the N-terminus and C-terminus of the target protein. The so-called 'Myc tag' and 'FLAG tag' epitopes, for example, are useful in this context. The epitope tags are useful in determining the extent to which the fusion protein is expressed and the extent to which the target protein is proteolytically removed. In particular, a population of yeast cells expressing the target protein fusion construct will have absolute levels of EGFR-Aga2 fusion protein that vary from cell to cell, but the ratio of antibodies binding to the target protein to antibodies binding to the distal epitope tag should be 1:1. In practice, the two antibodies are labeled with different fluorescent dyes, and the yeast cells are sorted with a FACS machine. Cells bearing a wild-type target protein, when detected by the FACS machine and sorted according to the two dyes, will distribute along a straight line that passes through the origin.

The yeast cells expressing an (epitope tagl)-EGFR-(epitope tag2)-Aga2 fusion protein are then mutagenized and then sorted. Cells in which the anti-EGFR antibody appears to not bind are selected, grown out, resorted and rechosen, and the process continued until yeast cells in which the anti-EGFR antibody appears not to bind constitute the majority of cells. Such cells are then plated to generate individual clones and then tested further and sequenced.

When the above procedure, as described in more detail in Cochran et al., *supra* and Chao et al., *supra,* was performed using an antibody of the invention such as EMD72000, yeast cells were isolated that express forms of EGFR with mutations in sites such as Asn452, Lys454, Phe457, Ser460, Gly461, Gln462, and/or Lys463 (see also Example 1).

Another method of identifying antibodies of the invention is to first generate a panel of antibodies against EGFR by standard methods such as hybridoma technology or phage display technology, and then screen the isolated antibodies so isolated for the ability to compete with EMD72000 in standard competition assays. Such methods are well known in the art of antibody engineering.

### Variable Region Sequence-Based Identification of Antibodies of the Invention

Based on our identification of Ser460/Gly461 as a part of the epitope for EMD72000, antibodies that have a variable region that is substantially identical to the variable region of EMD72000 are also considered useful antibodies in the methods of the invention. The variable region sequences of a candidate anti-EGFR antibody with an unknown epitope are aligned with variable region sequences of anti-EGFR antibodies with known epitopes, such as C225, EMD72000, MR1, and 806. This alignment can include the heavy chain variable region, the light chain variable region, the entire variable region, and the CDRs of the heavy chain or light chain or both. Figure 2 shows an alignment of several anti-EGFR antibody variable region sequences of known and unknown specificity. Based on this alignment, the h-R3 antibody is predicted to bind to the Ser460/Gly461 epitope, and is therefore considered useful in the invention.

### Function-Based Identification of Antibodies of the Invention

The method of treatment of the invention involves administering to a patient an antibody that blocks signaling by EGFR and EGFRvIII and similar receptors in which EGFR has a deletion in its extracellular domain. Therefore, methods for identifying antibodies useful in the invention also include the use of any assay that measures EGFR or EGFRvIII signaling in a mammalian cell line. Examples of such cell lines include either human mammary epithelial cells (HMECs), A431 cells, U87 cells, HT29 cells, or other cells, which have been transfected with an EGFRvIII expression construct. It is also possible to use mammalian cells that do not express EGFR and that have been transfected with an EGFRvIII expression construct. Signaling by EGFRvIII may be measured by a variety of mechanisms, such as those described below. *Measurement of Receptor Signaling by Determining Active Conformation*

The ability of an antibody of the invention to block signaling by EGFRvIII can be determined by its ability to prevent EGFRvIII from assuming a conformation that allows oligomerization. The extracellular domain of EGFR is thought to exist in two possible conformations: an active, signaling conformation corresponding to a dimeric structure seen by X-ray crystallography, and an inactive, non-signaling conformation corresponding to a monomeric X-ray structure (Garrett et al., Cell 110:763-773 (2002); Ferguson et al., Molecular Cell 11:507-517 (2003)). Schematic depictions of these two conformations are shown in Figure 1. The extracellular region of EGFR consists of four domains: domains 1 and 3 are helical beta-stranded structures, while domains 2 and 4 are elongated and rich in cysteines that form disulfide bonds. The amino acid sequences of domains 1 and 3 can be aligned, and the three-dimensional structures are similar. The conversion between the active and inactive forms occurs primarily by a rotation around arginine 310.

As an alternative method to identify antibodies of the invention, structural models of EGFRvIII in the active conformation can be used to identify regions which, when bound by an antibody, would result in steric inhibition of amino acids from about 274 to 310 from assuming an active conformation relative to domains 3 and 4. For example, antibodies recognizing amino acids from about 406 to 413 or recognizing amino acids from about 314 to 337 are useful in the methods of the invention.

Antibodies directed against such regions can, for example, be generated as follows. According to standard procedures, when such segments are normally bound by cysteines that form disulfide bonds, it is possible to use the disulfide-bonded loop, optionally conjugated to a carrier, as an antigen. For example, a disulfide-bonded loop with the sequence Cys-Asn-Gly-Ile-Gly-Ile-Gly-Glu-Phe-Lys-Asp-Ser-Leu-Ser-Ile-Asn-Ala-Thr-Asn-Ile-Lys-His-Phe-Lys-Asn-Cys (SEQ ID NO: 21), which corresponds to amino acids 313 to 338, may be used as an antigen to generate an antibody of the invention.

### Measurement of Receptor Signaling by Determining Receptor Internalization Rates

Dimerization and signaling by EGFRvIII can be measured by determining the internalization rates of antibody-EGFR complexes using methods known in the art for determining receptor internalization rates.

EGFRvIII signals constitutively, and in transfected human mammary epithelial cells the EGFRvIII mutant receptor has a high internalization rate as a result of endocytosis that is stimulated by signaling. Antibodies that bind to EGFRvIII in a way that inhibits signaling will also inhibit stimulated internalization, so that internalization takes place at a background level corresponding to bulk membrane turnover, which is about 10% of the rate of signaling-stimulated internalization. Conversely, antibodies that bind to EGFRvIII and do not inhibit its signaling will be internalized at a high rate. The extent of reduction or inhibition of receptor internalization can be at 10%, 20%, 30%, 40%, 50%, 60%, 70%, 80%, to 90%, or more, depending on the cell type used. This assay can also be used to examine the effects on signaling of intact EGFR, using cell lines that express only EGFR

In one example, the method of Worthylake is used (Worthylake et al., J. Biol. Chem. 274:8865-8874 (1999)) to measure rates of internalization. In brief, radiolabeled anti-EGFR antibodies are added to cultured mammalian cells expressing EGFRvIII, and optionally an approximately equal numbers of EGFR. At various times after the addition of antibody, aliquots of cells are removed and processed in either of two ways: an acid strip removes surface-bound antibody, which can be counted; and an alkaline hydrolysis releases internalized antibody, which can also be counted (see also Figures 4 and 5).

As described in more detail in the Examples, the monoclonal antibodies EMD72000 and MR1-1 do inhibit signaling by EGFRvIII. EMD72000 also inhibits signaling-stimulated internalization by intact EGFR. In other experiments, the non-neutralizing antibody 13A9 did not inhibit signaling-stimulated internalization by intact EGFR or EGFRvIII. MR1-1 does not bind to intact EGFR and so cannot be tested in this assay system.

### Measurement of receptor signaling by determining receptor kinase activity

Signaling by EGFRvIII, as well as by intact EGFR, can be measured by western blot using monoclonal antibodies that are specific for phosphorylated tyrosines at positions 1069 or 1173 (Upstate Biotechnology, Lake Placid, New York, catalog nos. 07-715 and 05-483, respectively) of EGFR. When cells expressing EGFRvIII, such as the cells listed above, are treated with an antibody recognizing the Ser460/Gly461 epitope, tyrosine phosphorylation of positions 1069, or 1173 is generally inhibited.

The extent of reduction or inhibition of receptor kinase activity can be at least 10%, 20%, 30%, 40%, 50%, 60%, 70%, 80%, 90%, or more, depending on the cell type used. In some cell types, a significant proportion of EGFRvIII has an intracellular location, such as in the endoplasmic reticulum or the Golgi, and is thus resistant to any antibody treatment.

### Use of the antibodies of the invention for the treatment of cancer

The antibodies of the invention are used to treat subjects having a cancer that are determined to express EGFRvIII. In one embodiment, the cancer can be assessed for the likelihood of expressing EGFRvIII based on clinical studies or published reports indicating the percent of a particular type of tumor that expresses EGFRvIII polypeptide or nucleic acid molecules in a given study. For example, a physician may note the tumor type and judge the percent likelihood that the patient's tumor expresses EGFRvIII, based on the scientific literature or clinical data.

For example, glial tumors, breast cancers, ovarian cancers, non-small cell lung carcinomas, and prostate carcinomas have been found to frequently express EGFRvIII (see for example Wikstrand et al., Cancer Res. 57:4130-4140 (1997)). Moscatello et al. (Cancer Res. 55:5536-5539 (1995)) reported that EGFRvIII was present in 57% (26 of 46) of high grade and 86% (6 of 7) of low grade glial tumors. EGFRvIII was not expressed in normal brain tissue. EGFRvIII was also found in 66% (4 of 6) of pediatric gliomas, 86% (6 of 7) of medulloblastomas, 78% (21 of 27) of breast carcinomas and 73% (24 of 32) ovarian cancers.

Garcia de Palazzo et al. (Cancer Res. 53:3217-3220 (1993)) used immunocytochemistry to determine EGFRvIII expression on 32 frozen sections of primary non-small cell lung cancer tumors. The mutation was identified in 16% of the specimens. For prostate carcinomas, 68% (26 of 3 8) of the specimens stained positive for EGFRvIII (Olapade-Olaopa et al., Brit. J. Cancer 82:186-194 (2000)).

Table 1, below, shows the percent likelihood of expressing EGFRvIII for a variety of cancers.

**Table 1. Prevalence of EGFRvIII Receptor in Various Carcinomas**

| **Cancer Type** | **% Expressing EGFRvIII** |
|---|---|
| Glial tumors (high grade) | 57% |
| Glial tumors (low grade) | 86% |
| Pediatric gliomas | 66% |
| Medulloblastomas | 86% |
| Breast carcinomas | 78% |
| Ovarian carcinomas | 73% |
| Non-small cell lung cancer | 16% |
| Prostate carcinomas | 68% |

If the subject of the invention has a cancer that is determined in a clinical study or published report to have a percent likelihood of expressing EGFRvIII that is at least 57%, 60%, 65%, 70%, 75%, 80%, 85%, 90%, 95%, or more, that cancer is considered in the methods of the invention to express EGFRvIII.

Alternatively or additionally, subjects are pre-tested for the expression of EGFRvIII polypeptides or nucleic acids, or similar extracellular deletion variants in their tumor(s). Diagnosis may be made by obtaining a tumor specimen and testing for EGFRvIII using protein, RNA, or DNA-based methods.

Protein-based methods, such as immunoassays or immunohistochemistry, can be used to test whether a tumor sample expresses EGFRvIII. For such assays, any EGFRvIII binding protein can be used, but it is preferable to use an antibody that recognizes the novel peptide junction sequence which includes the following amino acid sequence: Leu-Glu-Glu-Lys-Lys-Gly-Asn-Tyr-Val-Val-Thr-Asp-His (SEQ ID NO: 18), in which the Leu-Glu-Glu-Lys-Lys (SEQ ID NO: 19) sequence derives from exon 1 of EGFR, the glycine derives from the exon 1-exon 8 splice junction, and the Asn-Tyr-Val-Val-Thr-Asp-His (SEQ ID NO: 20) and any subsequent amino acids derive from exon 8. It should be noted that the novel peptide junction sequence can also include amino acids that are found in EGFR both at the amino terminus and carboxy terminus of SEQ ID NO: 18. Thus, an antibody that specifically recognizes the Lys-Gly-Asn junctional sequence and/or additional surrounding sequences may be used, provided that its non-specific binding is sufficiently low.

In one example, a tumor sample is obtained, for example, by biopsy or surgical removal. According to standard procedures, the tumor sample is then stained by immunohistochemical staining procedures. An antibody that specifically recognizes EGFRvIII is used as a primary antibody. For example, the variable regions of MR1-1 are attached to constant regions of an immunoglobulin such as from a mouse, rabbit or other animal for which secondary antibodies are commercially available. Such full-length antibodies are constructed by methods analogous to those described in Example 2. However, it is preferable to not use human constant regions, as the tumor tissue may contain human antibodies that would cross-react with a secondary antibody.

Alternatively, a polyclonal antisera that recognize the EGFRvIII novel peptide junction are obtained by standard methods, and then used for immunohistochemistry. For example, a synthetic peptide, such as Leu-Glu-Glu-Lys-Lys-Gly-Asn-Tyr-Val-Val-Thr-Asp-His-Gly-Cys (SEQ ID NO: 24) is obtained from a commercial custom peptide synthesis company. The C-terminal cysteine is used to conjugate the peptide to a carrier such as keyhole limpet hemocyanin, and then rabbits are immunized and boosted with the conjugate and appropriate adjuvant. After optional affinity purification of the anti-peptide antibodies, the resulting antiserum is used for immunoassays, such as immunohistochemistry, according to standard techniques known in the art.

Alternatively, expression of EGFRvIII may be tested using an RNA-based test. For example, a tumor sample can be tested using a reverse transcriptase-polymerase chain reaction (RT-PCR), a northern blot, RNAse protection assays, or quantitative real-time PCR, all of which are standard procedures. An RT-PCR procedure can be performed either on a purified RNA sample or by in situ methods, such as in situ hybridization or in situ reverse-transcriptase/polymerase chain reaction (RT/PCR) staining procedures.

For example, an oligonucleotide of about, for example, 20 to 30 bases that spans the EGFRvIII novel coding sequence junction may be used as a hybridization probe to detect EGFRvIII mRNA. The probe is designed to have the EGFRvIII novel peptide junction at its center, and the hybridization is performed under conditions such that the probe will hybridize only with EGFRvIII but not with intact EGFR mRNA. In some circumstances, it is useful to first perform a reverse-transcriptase/polymerase chain reaction step to amplify the EGFRvIII junction region. In addition to simply amplifying the signal, this step can be performed under conditions of rapid cycle time that lead to preferential amplification of short DNA segments, so that DNA segments corresponding to EGFRvIII are preferentially amplified compared to DNA segments corresponding to intact EGFR. Hybridization with a junction-spanning oligonucleotide probe is then performed on the amplified material.

In one variation of the method, one of the PCR primers corresponds to the novel peptide junction in EGFRvIII. The following 5' and 3' primers are exemplary primers that can be used in the method.
5' primer 5' GTCGGGCTCTGGAGGAAAAGAAAG GTAATTA 3' (SEQ ID NO: 13)
5' GAGTCGGGCTCTGGAGGAAAAGAAAG GTAA 3' (SEQ ID NO: 14)
3' primers 5' ATCCCAGTGGCGATGGACGGGATCT 3' (SEQ ID NO: 15)
5' GGTTTTCTGACCGGAGGTCCCAAACAGTTT 3' (SEQ ID NO: 16)

The underlined sequence represents the codon that corresponds to the glycine that results from a hybrid codon due to alternative splicing.

A DNA-based assay can also be used to detect EGFRvIII. This assay indicates the presence of genomic DNA that has undergone a deletion removing exons 2 through 7 and adjacent intronic material. This approach would generally involve a PCR-based method using oligonucleotide primers corresponding to regions within exon 1 and exon 8. This method is generally somewhat less preferred because in some cases it may be that EGFRvIII expression results from alternative splicing and not a genomic deletion. Moreover, even when EGFRvIII expression results from a genomic deletion, the endpoints of the deletion within the introns will vary from patient to patient, resulting in variation in signal intensity and PCR product size. A Southern blot may also be used to identify genomic deletions that lead to EGFRvIII expression, although surrounding normal tissue and the presence of a chromosome encoding wild-type EGFR will generally lead to difficulties in detection of the deleted DNA.

Once a cancer is identified as expressing EGFRvIII, one or more of the antibodies of the invention are administered to the subject to treat, stabilize, or prevent the cancer. The antibody can be administered in a dosage sufficient to cause a reduction in the size of a tumor, to slow or prevent an increase in the size of a tumor, to prevent an initial or subsequent occurrence of a tumor, to increase the disease-free survival time between the disappearance of a tumor and its reappearance, or to reduce an adverse symptom associated with a tumor.

The antibodies of the invention can also be used to treat a pre-cancerous lesion or prevent a tumor from developing in a subject determined to have a pre-cancerous lesion or biopsy sample that expresses EGFRvIII. Such a subject could be placed on a preventive regimen in order to reduce the likelihood of a malignancy from forming.

The precise dosage of antibodies of the invention for treatment or prevention of cancer will vary in accordance with factors appreciated by the typical clinician. These factors include (but are not limited to) size, age, overall health, the extent of the cancer, and other medications being administered to the patient. The development of a precise treatment regimen will require optimization through routine medical procedures well known to those in the medical arts.

The preferred route of administration of an antibody of the invention is intravenous, although intratumoral, intraperitoneal, subcutaneous, intramuscular, and intradermal injections may also be used, as well as other methods such as inhalation. A particularly preferred method of administration is intravenous infusion, for example over a period of about one hour.

The pharmaceutical compositions may be formulated according to conventional pharmaceutical practice (see, e.g., Remington: The Science and Practice of Pharmacy (20th ed.), ed. A.R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Liquid formulations of an antibody of the invention may include agents that stabilize proteins in solution, such as sugars, arginine or other amino acids, citrate, Tween, or human serum albumin. Phosphate-buffered saline may also be used as a liquid formulation.

Non-liquid formulations may also include the use of pharmaceutically acceptable excipients, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and anti-adhesives (e.g., magnesium stearate, zinc stearate, stearic acid, silicas, hydrogenated vegetable oils, or talc). Other pharmaceutically acceptable excipients can be, colorants, flavoring agents, plasticizers, humectants, buffering agents, and the like.

The preferred dose frequency is about once every 1 to 8 weeks, and more preferably about once every 1, 2, or 3 weeks. The preferred dose is about 200 to 2,000 milligrams for a 70 kg adult, more preferably about 400 to 1,200 milligrams, and most preferably about 800 milligrams per 70 kg adult. In one example, a 70-kilogram patient is infused with about 800 milligrams of EMD72000 about once every three to six weeks for about 3 to 6 cycles. The disease status of the patient is monitored, for example by radiological techniques, and treatment is continued if the first set of cycles is deemed successful. For additional information see Vanhoefer et al., J. Clin. Oncol. 22:175-184 (2004), Tabemero et al., Proc. Am. Soc. Clin. Oncol. 22:192 (2003), and Salazar et al., Proc. Am. Soc. Clin. Oncol. 23:127 (2004).

### Combination Therapies

The antibodies of the invention can be used in conjunction (e.g., before, during, or after) with additional anti-cancer therapies to prevent or reduce tumor growth or metastasis. Additional anti-cancer therapies include but are not limited to surgery, radiation therapy, chemotherapy, biologic therapy (e.g., cytokines, immunotherapy, and interferons), differentiating therapy, immune therapy, anti-angiogenic therapy, anti-metastatic therapy, inhibitors of EGFR such as antibodies that bind to distinct, non-overlapping epitopes as well as small molecule inhibitors of the EGFR kinase domain, non-steroidal antiinflammatory agents such as cyclo-oxygenase inhibitors, and hormone therapies. Non-limiting examples include paclitaxel, irinotecan, naproxen, aspirin, ibuprofen, and Velcade™. Examples of these additional anti-cancer therapies can be found, for example, in PCT Publication No. WO 2004/0323960. Antibodies of the invention may be formulated alone or in combination with any additional cancer therapies in a variety of ways that are known in the art.

### Examples

### Example 1. Identification of the epitope of EMD72000.

The epitope of EMD72000 on EGFR was identified essentially by the 'yeast display' method of Kieke et al. (U.S. Patent No. 6,300,065), Cochran et al., *supra,* and Chao et al., *supra* with the following specific variations. Chao et al. had found that the entire extracellular domain of EGFR was poorly expressed when fused to the Aga2 protein and expressed on the surface of the yeast *Saccharomyces cerevisiae,* so they fused amino acids 273-621 of EGFR to Aga2 for identification of the epitopes for the monoclonal antibodies 13A9, C225, and 806.

It was found that EMD72000 bound poorly to the EGFR (273-621)-Aga2 fusion protein on the surface of yeast, so a mutant derivative of the full-length EGFR extracellular domain was selected that was efficiently expressed on the surface of yeast as an Aga2 fusion protein. This mutant derivative has the amino acid substitutions Ala62Thr, Leu69His, Phe380Ser, and Ser418Gly.

The procedure of Chao et al. was then followed to identify EGFR mutations that disrupted EMD72000 binding, with the following modifications. In brief, a population of expression plasmids encoding the EGFR-Aga2 fusion protein was mutagenized and transformed into Saccharomyces cerevisiae according to standard procedures. The transformed yeast cells were put through four rounds of selection for EGFR mutants that had lost binding to EMD72000. The resulting population was expected to include mutants of EGFR that affected specific contact sites, as well as mutants of EGFR that destabilized the folded structure so that no antibody would properly bind. To eliminate mutants affecting the global structure of one or more domains of EGFR, two additional sortings were performed, selecting for yeast cells in which the antibody 225 would still bind to the EGFR on the cell surface (Figure 3). The antibody 225 was chosen because it had been previously established that 225 and 425 (from which EMD72000 was derived) bind to different epitopes on EGFR (Kreysch, PCT Patent Application Publication No. WO 2004/032960).

After these enrichments for yeast bearing mutant EGFRs, the EGFR sequences from 17 independent yeast clones were determined. All showed mutations corresponding to amino acid substitutions in EGFR amino acids 452-463. Table 2 shows representative specific substitutions. In some cases, multiple mutations were identified, but in all such cases there was a mutation at position 452, 454, 457, 460, 461, 462, or 463.

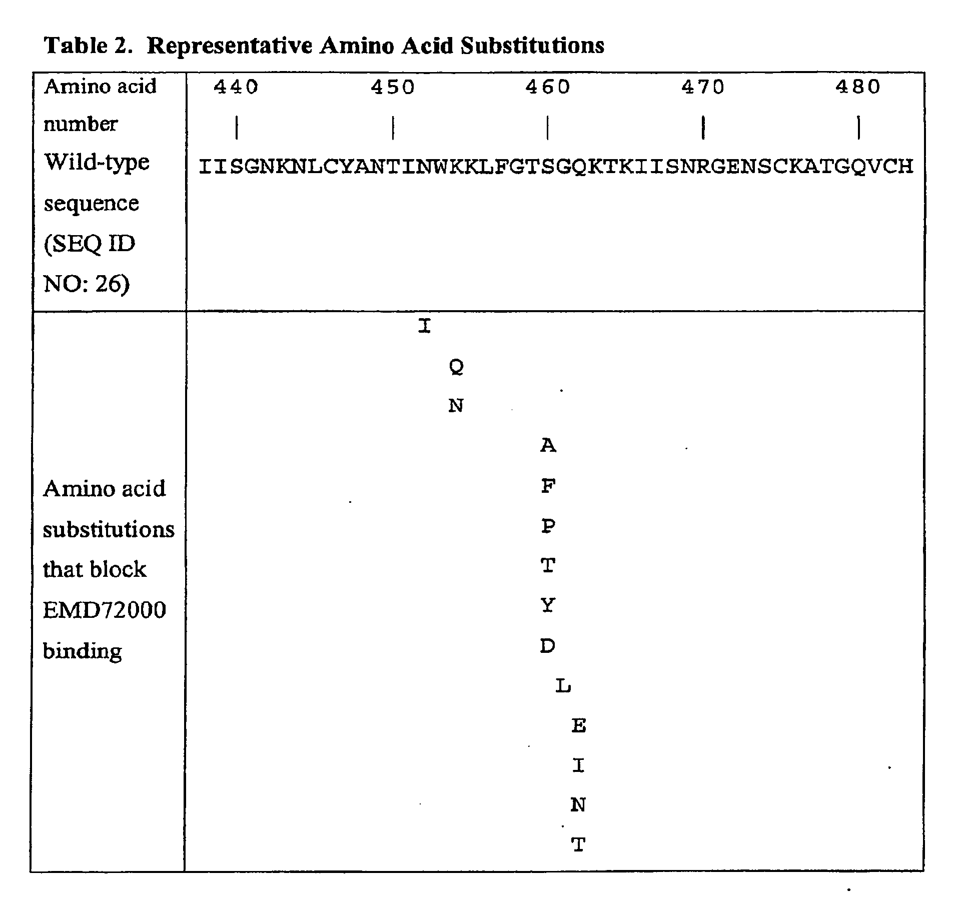

### Example 2. Construction of a full-length antibody that recognizes

### EGFRvIII.

MR1-1 refers to a set of variable regions that recognize the novel peptide junction in EGFRvIII. Beers et al. (Clin Cancer Res. 6:2835-43 (2000)) describe the optimization of these variable regions from the parental variable regions, termed MR1. Landry et al. (J Mol Biol. 308:883-93 (2001)) describe the solved structure of the MR1 variable regions with a peptide corresponding to the junctional peptide in EGFRvIII.

The variable regions of MR1-1 were placed in the context of an intact antibody with a human IgGI heavy chain and a kappa light chain for purposes of comparison with EMD72000 in the internalization studies in the following example.

The following standard methods were used to express MR1-1. DNA sequences encoding the heavy and light chain V region protein sequences of MR1-1 were inserted into the antibody expression vector pDHL 10, which is a derivative of pDHL2 (Gillies et al., J. Immunol. Methods 125:191-202 (1989)).

The following DNA sequence was used to encode the MR1-1 heavy chain V region:

CAGGTGAAGCTGCAGCAGTCTGGAGGAGGCTTGGTGAAGCCTGGAG
CTTCACTCAAACTCTCTTGTGTGACTTCTGGATTCACTTTCGCAAAT
TCGGAATGTCTTGGGTCCGCCAGACTTCTGACAAGTGTCTTGAGTGG
GTTGCTAGTATTAGTACAGGTGGTTACAACACCTACTATAGTGACAA
TGTGAAGGGGCGATTCACCATCTCCAGAGAGAATGCCAAGAACACC
CTGTACCTGCAAATGAGCAGTCTGAAATCTGAGGACACGGCCCTGT
ATTACTGTACGAGGGGGTATTCACCCTACTCATACGCTATGGACTAC
TGGGGTCAAGGAACCACCGTCACCGTCTCTGGATCGAGGGCCGCG.
(SEQ ID NO: 22)

The following DNA sequence was used to encode the MR1-1 light chain V region:

GACATTGAGGCTACACAGTCTCCTGCTTCCTTATCTGTAGCTACCGG

TGAGAAAGTTACTATCAGATGCATGACTAGCACTGACATTGATGAT
GATATGAACTGGTATCAACAGAAACCCGGTGAGCCACCCAAATTCC
TCATCTCCGAGGGAAACACTCTCAGGCCTGGGGTTCCGTCCCGCTTT
AGTAGTAGTGGGACTGGGACAGACTTCGTTTTCACCATCGAGAACA
CTCTCTCCGAGGACGTGGGGGATTACTACTGCTTGCAGTCCTGGAAC
GTCCCATTAACATTCGGCTGCGGACAAAGTTGGAAATAAAAC.
(SEQ ID NO: 23)

Electroporation was used to introduce the DNA encoding the MR1-1 intact antibody described above into a mouse myeloma NS/0 cell line. To perform electroporation, cells were grown in Dulbecco's modified Eagle's medium supplemented with 10% heat-inactivated fetal bovine serum, 2 mM glutamine and penicillin/streptomycin. About 5x10⁶ cells were washed once with PBS and resuspended in 0.5 ml PBS. 10 µg of linearized plasmid DNA encoding the antibody with MR1-1 V regions and human IgG1 constant regions was then incubated with the cells in a Gene Pulser Cuvette (0.4 cm electrode gap, BioRad) on ice for about 10 minutes. Electroporation was performed using a Gene Pulser (BioRad, Hercules, CA) with settings at 0.25 V and 500 µF. Cells were allowed to recover for about 10 minutes on ice, after which they were resuspended in growth medium and plated onto two 96 well plates.

Stably transfected clones were selected by their growth in the presence of 100 nM methotrexate (MTX), which was added to the growth medium two days post-transfection. The cells were fed two to three more times on every third day, and MTX-resistant clones appeared in 2 to 3 weeks. Supernatants from clones were assayed by anti-Fc ELISA to identify clones that produced high amounts of the anti-GD2 antibody. High producing clones were isolated and propagated in growth medium containing 100 nM MTX. Typically, a serum-free growth medium, such as H-SFM or CD medium (Life Technologies), was used.

### Example 3. Inhibition of EGFRvIII signaling by an antibody that binds to

### the Ser460/Gly461 epitope.

The ability of various antibodies to inhibit signaling by EGFR and EGFRvIII was tested using four cell lines derived from human mammary epithelial cells (HMECs), whose properties are described in Table 3 below. The parental HMECs were obtained from the American Type Culture Collection (Manassas, VA USA). Cells expressing EGFRvIII were derived from the parental HMEC line by viral transduction with an EGFRvIII expressing virus constructed according to standard procedures (see, for example, Nishikawa et al., Proc. Natl. Acad. Sci. 91:7727-7731 (1994). After recovery from the viral transduction protocol and some outgrowth, successfully infected cells were sorted using a FACS machine into populations expressing high, medium and low levels of EGFRvIII.

**Table 3. Inhibition of Receptor Signaling**

| Cell line | EGFR+ expression level | EGFRvIII expression level |
|---|---|---|
| Parental HMECs (pHMECs) | 2x10⁵/cell | 0 |
| HMEC-vIII low | 2x10⁵/cell | 5x10⁴/cell |
| HMEC-vIII medium | 2x10⁵/cell | 2x10⁵/cell |
| HMEC-vIII high | 2x10⁵/cell | 6x10⁵/cell |

The rationale for using HMECs was as follows. EGFR plays an important role in normal breast development, and is expressed at high levels in these cells. In addition, the mechanisms for receptor-mediated endocytosis, recycling, and degradation operate at a high level in these cells.

To measure the effect on EGFR and EGFRvIII signaling of a given antibody binding to EGFR or EGFRvIII, radiolabeled antibodies were added to cultures of the HMECs described in the table above. The background, rationale and design for this experiment were as follows. Normally, when EGFR molecule signals, it is actively endocytosed as a result of activation of intracellular signaling pathways. This endocytosis happens on the order of a few minutes, with an internalization rate of up to 15% per minute or more. In the absence of EGFR signal transduction, EGFR is internalized slowly as part of bulk membrane movement, with an internalization rate of about 2% per minute. When a non-neutralizing radiolabeled antibody binds to EGFR on a mammalian cell, the antibody will be internalized at a high rate if a ligand is present or if the EGFR is constitutively signaling for some other reason. When a neutralizing radiolabeled antibody binds to EGFR, signaling will stop and signaling-stimulated endocytosis will also stop. As a result, a radiolabeled neutralizing antibody bound to EGFR will be internalized at a slow rate of about 2% per minute.

To measure internalization rates, the method of Worthylake et al., *supra* was used, as schematically depicted in Figure 4. In brief, radiolabelled antibodies were added to culture parental HMECs and to HMECs expressing EGFRvIII. At times of 0, 1, 2, 3, 5 and 10 minutes, cell samples were withdrawn, washed, and then acid-stripped to remove and measure surface-bound radiolabeled antibody. The cells were then lysed and the remaining radiolabel was measured to determine levels of internalized antibody. An internalization rate was calculated according to the formulas in Figure 5.

The data shown in Figure 6 were obtained with radiolabeled EGF, a EMD72000, and MR1-1. In separate experiments, data were obtained with the non-neutralizing antibody 13A9. These data indicated that radiolabeled EGF, when bound to the surface of any of the four cell lines, was rapidly internalized at a rate kₑ of about 0.14/minute; i.e. the instantaneous rate of internalization of EGF was about 14% per minute.

When radiolabeled 13A9 was added to the parental HMEC line, its internalization rate in the range of 0.025/minute, a low internalization rate characteristic of bulk membrane internalization. In contrast, when EGF was also present, radiolabeled 13A9 was internalized at a high rate that was similar to the rate of radiolabeled EGF internalization. These observations are consistent with the description of 13A9 as a non-neutralizing antibody with respect to EGF binding to EGFR.

Also in contrast, in the HMEC lines expressing EGFRvIII, radiolabeled 13A9 was internalized at a high rate in the presence and absence of EGF. These observations are consistent with the idea that EGFRvIII is able to signal and internalize in a manner that is constitutive and ligand-independent.

When radiolabeled EMD72000 was bound to parental HMECs expressing only EGFR in the presence of EGF, the internalization rate was low, indicating that EMD72000 inhibits signal transduction from intact EGFR. In addition, when radiolabeled EMD72000 was bound to HMECs that express EGFRvIII as well as intact EGFR, the internalization rate was low, indicating that EMD72000 inhibited the signaling due to EGFRvIII as well as intact EGFR.

The antibody MR1-1 binds to the novel junction peptide within EGFRvIII. Specifically, MR1-1 recognizes the sequence Lys-Lys-Gly-Asn-Tyr-Val-Val-Thr-Asp-His (SEQ ID NO: 25), which is expressed from the juxtaposition of Exon 1 and Exon 8 of EGFR. MR1-1 does not bind to intact EGFR expressed on the surface of cells. Radiolabeled MR1-1 is internalized slowly by HMECs expressing both EGFRvIII and intact EGFR. Without wishing to be bound by theory, it seems likely that when cells expressing intact EGFR and EGFRvIII are treated with radiolabeled MR1-1, this protein binds only to EGFRvIII and inhibits the signaling through those receptors to which it is bound, but does not bind to intact EGFR, so that signaling through EGFR homodimers will not be inhibited.

### Example 4. Structure-based prediction of antibody inhibition of EGFR and EGFRvIII signaling.

To understand the possible structural basis of inhibition of EGFRvIII signaling by EMD72000 and to fully illustrate methods for obtaining antibodies that inhibit signaling of both EGFRvIII and intact EGFR, the following structural models were constructed:
Model 1. Intact EGFR/EGFRvIII heterodimers in the signaling conformation.
Model 2. EGFRvIII homodimers in the signaling conformation.
Model 3. An Fab docked in the region of the Ser460/Gly461 epitope on a model of EGFRvIII in an active conformation.
Model 4. An Fab docked in the region of the Ser460/Gly461 epitope on a model of EGFRvIII in an inactive conformation.

Images of these models are shown in Figures 7-9. Those skilled in the art of protein modeling will recognize that, to fully appreciate such structural models, it is best to view such models using a viewer program such as RasMol (Sayle RA and Milner-White EJ, Trends Biochem. Sci. 20:374 (1995)) or SwissPDBViewer (Kaplan et al. Brief Bioinform. 2:195-7 (2001)) or other similar programs, which allow the viewer to rotate, translate, and change the size of the viewed model. In addition, particular features of a model such as important amino acids and polypeptide chains may be colored and/or labeled by the user to highlight features of interest.

The models were constructed using SwissPDBViewer as follows. To construct Model 1 of an intact EGFR/EGFRvIII heterodimer, amino acids 1-273 were deleted from one copy of EGFR in the structure file 1MOX (Garrett TP, et al., Cell 110:763-73 (2002)). To construct Model 2 of an EGFRvIII/EGFRvIII homodimer, amino acids 1-273 were deleted from both copies of EGFR in the structure file 1MOX. No attempt was made to model amino acids 1 through 7 as fused to amino acid 274, as amino acids 1-7 were hypothesized to be either disordered in EGFRvIII or cleaved at the Lys/Lys sequence.

Based on models 1 and 2 (Figure 7), it was apparent that the remnant of domain 2 in EGFRvIII, which includes amino acids from about 274 to about 310, makes significant dimer contacts and most likely makes a favorable energetic contributions to formation of intact EGFR/EGFRvIII heterodimers and EGFRvIII/EGFRvIII homodimers. Such a favorable energetic contribution is most likely only possible when the domain 2 remnant in EGFRvIII is in the correct conformation, which according to a theory of the invention corresponds to the same conformation that amino acids 274-310 have in the active dimer of intact EGFR.

Model 3 (Figure 8) was constructed from Model 1 by the addition of an Fab moiety, which was roughly docked to the protrusion of Ser460 and Gly461 in EGFRvIII. The result of this model-building exercise indicated that the positioning of an Fab fragment over the Ser460/Gly461 epitope indicated that the Fab would sterically collide with EGFR amino acids 274-310 in the active signaling conformation. However, such an Fab would not sterically collide with EGFR amino acids 274-310 in the inactive, non-signaling conformation seen, for example, in the structure of Model 4. Mode 4 was constructed by placement of the Fab moiety as positioned in Model 3 into a structure that has EGFR in the inactive conformation.

Without wishing to be bound by theory, these results appear to explain the observations of Example 2, namely that EMD72000 binding to EGPRvIII inhibited the signaling of EGFRvIII.

In addition, these observations illustrate a method of the invention,
wherein antibodies of the invention are identified by comparing models of the three-dimensional structures of EGFRvIII in active and inactive states, finding surfaces that are hidden in the active state and exposed in the inactive state, and then identifying antibodies that bind on or near such surfaces. The models of EGFRvIII in the active and inactive states are usually generated by removing amino acids 1-273 from models of the solved structures of EGFR in the active and inactive states. Antibodies that bind to the desired surfaces are identified, for example, by first identifying anti-EGFR antibodies with distinct epitopes, and then identifying the epitopes of the various antibodies, for example by yeast display.

### Other Embodiments

From the foregoing description, it will be apparent that variations and modifications may be made to the invention described herein to adopt it to various usages and conditions. Such embodiments are also within the scope of the following claims.

All publications, patent applications, and patents mentioned in this specification, including US Provisional Application No. 60/788,426, filed March 31, 2006, are herein incorporated by reference to the same extent as if each independent publication, patent, or patent application was specifically and individually indicated to be incorporated by reference.

The following pages 44 to 49 contain specific embodiments.
1. A method for treating, preventing, or stabilizing a cancer in a subject in need thereof, said method comprising the steps of:
   (a) determining if said cancer expresses EGFRvIII; and
   (b) administering to said subject, determined in step (a) to have a cancer that expresses EGFRvIII, an antibody that recognizes the Ser460/Gly461 epitope of human EGFR,
   wherein said antibody is administered for a time and in an amount sufficient to treat, prevent, or stabilize said cancer in said subject.
2. The method of; 1, wherein said cancer is selected from the group consisting of glioblastoma, medulloblastoma, breast cancer, ovarian cancer, and prostate carcinoma.
3. The method of 1, wherein said determining of step (a) comprises a comparison between the type of cancer in said subject and the known percent incidence of EGFRvIII for the same type of cancer in a population to determine the percent likelihood that said cancer expresses EGFRvIII.
4. The method of 3, wherein said percent likelihood is at least 57%.
5. The method of 1, wherein said determining of step (a) comprises obtaining a biological sample from said subject and detecting an EGFRvIII polypeptide in said biological sample, wherein said detecting comprises the use of an EGFRvIII binding polypeptide or an anti-EGFRvIII antiserum.
6. The method of 5, wherein said EGFRvIII binding polypeptide is an antibody that specifically recognizes the EGFRvIII novel peptide junction.
7. The method of 1, wherein said determining of step (a) comprises obtaining a biological sample from said subject and detecting an EGFRvIII nucleic acid in said biological sample, wherein said detecting comprises the use of a nucleic acid that hybridizes to a nucleic acid sequence that encodes the EGFRvIII mRNA novel peptide junction, or its corresponding cDNA.
8. The method of 1, wherein said determining of step (a) comprises obtaining a biological sample from said subject and detecting an EGFRvIII nucleic acid in said biological sample, wherein said detecting comprises the use of a nucleic acid that hybridizes to an EGFR nucleic acid and a nucleic acid that hybridizes to an EGFRvIII nucleic acid, wherein said detecting comprises distinguishing between the size of said EGFRvIII nucleic acid and said EGFR nucleic acid.
9. The method of 8, wherein said distinguishing comprises the use of an amplification based assay, Southern blot, northern blot, or RNase protection assay.
10. The method of 9, wherein said amplification based assay is PCR, RT-PCR, or quantitative real time PCR.
11. The method of 1, wherein said antibody comprises a heavy chain variable region amino acid sequence that is at least 90% identical to the amino acid sequence of SEQ ID NO: 9.
12. The method of 1, wherein said antibody comprises a light chain variable region amino acid sequence that is at least 90% identical to the amino acid sequence of SEQ ID NO: 3.
13. The method of 12, wherein said antibody comprises a variable region amino acid sequence that is at least 90% identical to the amino acid sequence of SEQ ID NOs: 3 and 9.
14. The method of 1, wherein said antibody is EMD72000, mAb528, h-R3, mAB 425, antigen binding fragments thereof, humanized, or chimeric derivatives thereof.
15. The method of 14, wherein said antibody is EMD72000.
16. The method of 1, wherein said antibody reduces or prevents signaling by intact EGFR or EGFRvIII.
17. The method of 16, wherein said signaling by intact EGFR or EGFRvIII comprises conversion to active conformation, receptor internalization, receptor dimerization, receptor autophosphorylation, and phosphorylation of a substrate.
18. The method of 17, wherein said antibody reduces signaling by EGFRvIII by at least 20%.
19. The method of 1, wherein said subject has a pre-cancerous lesion that is determine in step (a) to express said EGFRvIII and said method is used to prevent cancer in said subject.
20. A method of treating, preventing, or stabilizing a cancer in a subject in need thereof, said method comprising the steps of:
   (a) determining if said cancer expresses an EGFRvIII; and
   (b) administering to said subject, determined in step (a) to have a cancer that expresses EGFRvIII, an antibody that binds EGFRvIII and reduces or inhibits receptor signaling by EGFRvIII,
   wherein said antibody is administered for a time and in an amount sufficient to treat, prevent, or stabilize said cancer in said subject.
21. The method of 20, wherein said cancer is selected from the group consisting of glioblastoma, medulloblastoma, breast cancer, ovarian cancer, and prostate carcinoma.
22. The method of 20, wherein said determining of step (a) comprises a comparison between said cancer in said subject and the known percent incidence of EGFRvIII for the same type of cancer to determine the percent likelihood that said cancer expresses EGFRvIII.
23. The method of 22, wherein said percent likelihood is at least 57%.
24. The method of 20, wherein said determining of step (a) comprises obtaining a biological sample from said subject and detecting an EGFRvIII polypeptide in said biological sample, wherein said detecting comprises the use of a EGFRvIII binding polypeptide or anti-EGFRvIII antiserum.
25. The method of 24, wherein said EGFRvIII binding polypeptide is an antibody that specifically recognizes the EGFRvIII novel peptide junction.
26. The method of 20, wherein said determining of step (a) comprises obtaining a biological sample from said subject and detecting an EGFRvIII nucleic acid in said biological sample, wherein said detecting comprises the use of a nucleic acid that hybridizes to a nucleic acid sequence that encodes the EGFRvIII mRNA novel peptide junction, or its corresponding cDNA.
27. The method of 20, wherein said determining of step (a) comprises obtaining a biological sample from said subject and detecting an EGFRvIII nucleic acid in said biological sample, wherein said detecting comprises the use of a nucleic acid that hybridizes to an EGFR nucleic acid and a nucleic acid that hybridizes to an EGFRvIII nucleic acid, wherein said detecting comprises distinguishing between the size of said EGFRvIII nucleic acid and said EGFR nucleic acid.
28. The method of 27, wherein said distinguishing comprises the use of an amplification based assay, Southern blot, northern blot, or RNase protection assay.
29. The method of 28, wherein said amplification based assay is PCR, RT-PCR, or quantitative real time PCR.
30. The method of 20, wherein said antibody binds to an epitope comprising Asn 452, Lys454, Phe457, Ser460, Gly461, Gln462, or Lys463 of human EGFR, amino acids 406 to 413 of human EGFR, or amino acids 314 to 337 of human EGFR, or any combination thereof.
31. The method of 20, wherein said antibody comprises a heavy chain variable region amino acid sequence that is at least 90% identical to the amino acid sequence of SEQ ID NO: 9.
32. The method of 20, wherein said antibody comprises a light chain variable region amino acid sequence that is at least 90% identical to the amino acid sequence of SEQ ID NO: 3.
33. The method of 32, wherein said antibody comprises a variable region amino acid sequence that is at least 90% identical to the amino acid sequence of SEQ ID NOs: 3 and 9.
34. The method of 20, wherein said antibody is EMD72000.
35. The method of 20, wherein said receptor signaling comprises conversion to active conformation, receptor internalization, receptor dimerization, receptor autophosphorylation, or phosphorylation of a substrate.
36. The method of 35, wherein said receptor signaling is receptor internalization.
37. The method of 35, wherein said receptor signaling is receptor dimerization.
38. The method of 35, wherein said antibody inhibits EGFRvIII from adopting an active conformation.
39. The method of 35, wherein said antibody reduces the receptor signaling by intact EGFR by at least 20% and by EGFRvIII by at least 20%.

## Claims

1. A method for identifying an anti-epidermal growth factor receptor (EGFR) antibody that reduces or prevents signaling by intact EGFR or EGFRvIII, said method comprising:
a) contacting a candidate antibody with an EGFR protein, or fragment thereof, having a wild-type serine at position 460 and a wild-type glycine at position 461;
b) contacting said candidate antibody with an EGFR protein, or fragment thereof, having a mutation at positions 460 and 461; and
c) comparing the binding of the candidate antibody to the EGFR protein, or fragment thereof, in step (a) with the binding of the candidate antibody to the EGFR protein, or fragment thereof, in step (b), wherein an antibody that binds to the EGFR of step (a) but not step (b) is identified as an anti-EGFR antibody that reduces or prevents signaling by intact EGFR or EGFRvIII.

2. The method of claim 1, wherein said mutation at amino acid 460 is a mutation of serine to alanine, phenylalanine, proline, threonine, tyrosine, or aspartic acid.

3. The method of claim 1 or 2, wherein said mutation at amino acid 461 is a mutation of glycine to leucine.

4. The method of any one of claims 1 to 3, wherein said fragment of EGFR comprises amino acids 273-621 of EGFR.

5. The method of claim 4, wherein said fragment of EGFR further comprises one or more amino acid substitutions selected from the group consisting of alanine to threonine at amino acid 62, leucine to histidine at amino acid 69, phenylalanine to serine at amino acid 380, and serine to glycine at amino acid 418.

6. The method of any one of claims 1 to 5, wherein said epitope further comprises at least one additional amino acid selected from the group consisting of amino acids 452, 454, 457, 462, and 463 of EGFR and wherein said EGFR protein, or fragment thereof, of step (a) further comprises a wild type amino acid at positions 452, 454, 457, 462, and 463 of EGFR and said EGFR protein of step (b) further comprises a mutation in one or more amino acid at positions 452, 454, 457, 462, or 463, wherein an antibody that binds to the EGFR of step (a) but not step (b) is identified as an anti-EGFR antibody that binds to an epitope comprising Ser460 and Gly461 and at least one additional amino acid selected from the group consisting of amino acids 452, 454, 457, 462, and 463 of EGFR.

7. The method of any one of claims 1 to 6, wherein said fragment of EGFR is a fragment that comprises the extracellular domain of EGFR or EGFRvIII.

8. The method of claim 7, wherein said fragment of EGFR consists of the extracellular domain of EGFR or EGFRvIII.

9. A method for identifying an anti-EGFR antibody that reduces or prevents signaling by intact EGFR or EGFRvIII, said method comprising:
a) contacting an EGFR or EGFRvIII polypeptide, or fragment thereof, with EMD72000;
b) measuring the binding of said EMD72000 to said EGFR or EGFRvIII polypeptide, or fragment thereof;
c) contacting said EGFR or EGFRvIII polypeptide, or fragment thereof, and EMD72000 of step (a) with a candidate antibody; and
d) measuring the binding of said EMD72000 to said EGFR or EGFRvIII polypeptide, or fragment thereof, wherein a decrease in the binding of said EMD72000 to said EGFR or EGFRvIII as compared to the binding measured in step (b) identifies said candidate antibody as an antibody that reduces or prevents signaling by intact EGFR or EGFRvIII.

10. A method for identifying an anti-EGFR antibody that reduces or prevents signaling by intact EGFR or EGFRvIII, said method comprising:
a) contacting a candidate antibody with a fragment of EGFR comprising amino acids 314-337 or 406-413 of EGFR protein;
b) determining the binding of said candidate antibody to said fragment of EGFR, wherein an antibody that binds to said fragment of EGFR of step (a) is identified as an anti-EGFR antibody that reduces or prevents signaling by intact EGFR or EGFRvIII.

11. The method of claim 10, wherein said fragment of EGFR comprises SEQ ID NO: 21.

12. The method of any one of claims 1 to 11, wherein said signaling by intact EGFR or EGFRvIII comprises conversion to active conformation, receptor internalization, receptor dimerization, receptor autophosphorylation, and/or phosphorylation of a substrate.

13. The method of claim 12, wherein said signaling by intact EGFR or EGFRvIII is receptor dimerization.
